# EUROPEAN PATENT APPLICATION

(11) **EP 2 871 244 A1**
(43) Date of publication of application: **13.05.2015**
(21) Application number: 13812618.0
(22) Date of filing: 03.07.2013
(51) Int. Cl.: C12Q 1/68, C12M 1/00, C12M 1/34

(54) **NUCLEIC ACID CHROMATOGRAPHY METHOD, COMPOSITION FOR NUCLEIC ACID CHROMATOGRAPHY, AND KIT CONTAINING SAME**

(30) Priority: 05.07.2012 JP 2012151755
(71) Applicant: NGK Insulators, Ltd., Nagoya-shi, Aichi 467-8530 (JP)
(72) Inventor: ORIBE, Akinobu, Nagoya-shi Aichi 467-8530 (JP); NIWA, Kousuke, Nagoya-shi Aichi 467-8530 (JP); HIROTA, Toshikazu, Nagoya-shi Aichi 467-8530 (JP)
(74) Representative: Naylor, Matthew John
(86) International application number: PCT/JP2013/068251
(87) International publication number: WO 2014/007289

(57) **Abstract**

Provided is a nucleic acid chromatography method ensuring a stable detection state, and a composition and kit for use in nucleic acid chromatography. For this purpose, the nucleic acid chromatography method is provided with a step of performing chromatography by supplying a chromatography liquid containing a target nucleic acid to a solid-phase carrier on which is fixed a detection probe capable of capturing the nucleic acid, wherein the chromatography liquid contains one or two or more kinds of water-soluble polymers.

### [Sequence List]

SEQUENCE LISTING

<110>  NGK INSULATORS, LTD.

<120>  Method of Nucleotide Chromatograpy

<130>  K12-357-PCT

<160>  103

<170>  PatentIn version 3.5

<210>  1
<211>  23
<212>  DNA
<213>  Artificial

<220>
<223>  probe

<400>  1
tgttctctga ccaatgaatc tgc                                              23

<210>  2
<211>  23
<212>  DNA
<213>  Artificial

<220>
<223>  probe

<400>  2
tggaactggg aacgctttag atg                                              23

<210>  3
<211>  23
<212>  DNA
<213>  Artificial

<220>
<223>  probe

<400>  3
ttcgcttcgt tgtaatttcg gac                                              23

<210>  4
<211>  23
<212>  DNA
<213>  Artificial

<220>
<223>  probe

<400>  4
aggcatccta agaaatcgct act                                              23

<210>  5
<211>  23
<212>  DNA

<213>  Artificial

<220>
<223>  probe

<400>  5
tagcccagtg atttatgaca tgc                                              23


<210>  6
<211>  23
<212>  DNA
<213>  Artificial

<220>
<223>  probe

<400>  6
cgctctggtt actattggac gtt                                              23


<210>  7
<211>  23
<212>  DNA
<213>  Artificial

<220>
<223>  probe

<400>  7
tagccaactc taaataacgg acg                                              23


<210>  8
<211>  23
<212>  DNA
<213>  Artificial

<220>
<223>  probe

<400>  8
ttcggttgtc gatatgagga tct                                              23


<210>  9
<211>  23
<212>  DNA
<213>  Artificial

<220>
<223>  probe

<400>  9
   ggggggtact tcatacaaga tgc                                               23


<210>  10
<211>  23
<212>  DNA
<213>  Artificial

<220>

<223>  probe

<400>  10
gagtagcagg caaataccct aga                                              23


<210>  11
<211>  23
<212>  DNA
<213>  Artificial

<220>
<223>  probe

<400>  11
gcctattaag gtctacgtca tcg                                              23


<210>  12
<211>  23
<212>  DNA
<213>  Artificial

<220>
<223>  probe

<400>  12
agtcatacag tgaggaccaa atg                                              23


<210>  13
<211>  23
<212>  DNA
<213>  Artificial

<220>
<223>  probe

<400>  13
cattcgacat aagctgttga tgc                                              23


<210>  14
<211>  23
<212>  DNA
<213>  Artificial

<220>
<223>  probe

<400>  14
tgctcactta cattacgtcc atg                                              23


<210>  15
<211>  23
<212>  DNA
<213>  Artificial

<220>
<223>  probe

<400>  15

tacacctatc aactcgtaga gca                                              23

<210>  16
<211>  23
<212>  DNA
<213>  Artificial

<220>
<223>  probe

<400>  16
aggtccggta gtaatttagg tgc                                              23

<210>  17
<211>  23
<212>  DNA
<213>  Artificial

<220>
<223>  probe

<400>  17
tgcactctga tatatacagg cca                                              23

<210>  18
<211>  23
<212>  DNA
<213>  Artificial

<220>
<223>  probe

<400>  18
gcagccctta tagataacgg gac                                              23

<210>  19
<211>  23
<212>  DNA
<213>  Artificial

<220>
<223>  probe

<400>  19
gaagccatga tactgttcag ggt                                              23

<210>  20
<211>  23
<212>  DNA
<213>  Artificial

<220>
<223>  probe

<400>  20
tattctacca acgacatcac tgc                                              23

<210>  21
<211>  23
<212>  DNA
<213>  Artificial

<220>
<223>  probe

<400>  21
ccatcagtta ttcggaggga ctc                                               23


<210>  22
<211>  23
<212>  DNA
<213>  Artificial

<220>
<223>  probe

<400>  22
ccatatccga ttattagcga cgg                                               23


<210>  23
<211>  23
<212>  DNA
<213>  Artificial

<220>
<223>  probe

<400>  23
catctccaag aattgaccca cca                                               23


<210>  24
<211>  23
<212>  DNA
<213>  Artificial

<220>
<223>  probe

<400>  24
ccgtcgtgtt attaaagacc cct                                               23


<210>  25
<211>  23
<212>  DNA
<213>  Artificial

<220>
<223>  probe

<400>  25
gaaggatcgc ttttatctgg cat                                               23


<210>  26
<211>  23
<212>  DNA

<213>  Artificial

<220>
<223>  probe

<400>  26
catttgtcag gtacagtcca ctt                                              23


<210>  27
<211>  23
<212>  DNA
<213>  Artificial

<220>
<223>  probe

<400>  27
gcccacactc ttacttatcg act                                              23


<210>  28
<211>  23
<212>  DNA
<213>  Artificial

<220>
<223>  probe

<400>  28
cgctgttact gtaagcgtac tag                                              23


<210>  29
<211>  23
<212>  DNA
<213>  Artificial

<220>
<223>  probe

<400>  29
cgcgattcct attgattgat ccc                                              23


<210>  30
<211>  23
<212>  DNA
<213>  Artificial

<220>
<223>  probe

<400>  30
ccgtctgggt taaagattgc tag                                              23


<210>  31
<211>  23
<212>  DNA
<213>  Artificial

<220>

<223>  probe

<400>  31
agtcagtcca aatctcagga tgg                                              23


<210>  32
<211>  23
<212>  DNA
<213>  Artificial

<220>
<223>  probe

<400>  32
cgcctaaatg aaactcactc tgc                                              23


<210>  33
<211>  23
<212>  DNA
<213>  Artificial

<220>
<223>  probe

<400>  33
ggggtcaaac caacaattga tct                                              23


<210>  34
<211>  23
<212>  DNA
<213>  Artificial

<220>
<223>  probe

<400>  34
gcccattgat agaattacga ggc                                              23


<210>  35
<211>  23
<212>  DNA
<213>  Artificial

<220>
<223>  probe

<400>  35
atgccgttgt caagagttat ggt                                              23


<210>  36
<211>  23
<212>  DNA
<213>  Artificial

<220>
<223>  probe

<400>  36

tgccggctat cgtaagtata tgc                                              23


<210>  37
<211>  23
<212>  DNA
<213>  Artificial

<220>
<223>  probe

<400>  37
gcacctcata ccttcataga gca                                              23


<210>  38
<211>  23
<212>  DNA
<213>  Artificial

<220>
<223>  probe

<400>  38
cgcgacattt agtccaggag atg                                              23


<210>  39
<211>  23
<212>  DNA
<213>  Artificial

<220>
<223>  probe

<400>  39
ctagtccatt gtaacgaagg cca                                              23


<210>  40
<211>  23
<212>  DNA
<213>  Artificial

<220>
<223>  probe

<400>  40
agacaattag aatcagtgcc cct                                              23


<210>  41
<211>  23
<212>  DNA
<213>  Artificial

<220>
<223>  probe

<400>  41
gcattgaggt attgttgctc cca                                              23

<210>  42
<211>  23
<212>  DNA
<213>  Artificial

<220>
<223>  probe

<400>  42
cgagagtctg taatagccga tgc                                               23


<210>  43
<211>  23
<212>  DNA
<213>  Artificial

<220>
<223>  probe

<400>  43
tgccgtgata cttaactacg cta                                               23


<210>  44
<211>  23
<212>  DNA
<213>  Artificial

<220>
<223>  probe

<400>  44
gagtccgcaa aaatatagga ggc                                               23


<210>  45
<211>  23
<212>  DNA
<213>  Artificial

<220>
<223>  probe

<400>  45
gcctcacata actggagaaa cct                                               23


<210>  46
<211>  23
<212>  DNA
<213>  Artificial

<220>
<223>  probe

<400>  46
cgccaatgac aataagttga ggc                                               23


<210>  47
<211>  23
<212>  DNA

<213>  Artificial

<220>
<223>  probe

<400>  47
cgcgatataa cattaaccga ggc                                              23


<210>  48
<211>  23
<212>  DNA
<213>  Artificial

<220>
<223>  probe

<400>  48
cacgcttagt tcctacctta ggc                                              23


<210>  49
<211>  23
<212>  DNA
<213>  Artificial

<220>
<223>  probe

<400>  49
cgcgtcgaat tacttaatca cca                                              23


<210>  50
<211>  23
<212>  DNA
<213>  Artificial

<220>
<223>  probe

<400>  50
gggataggta ttatgctcca gcc                                              23


<210>  51
<211>  23
<212>  DNA
<213>  Artificial

<220>
<223>  probe

<400>  51
cgccattata caacggttca tgc                                              23


<210>  52
<211>  23
<212>  DNA
<213>  Artificial

<220>

<223>  probe

<400>  52
gcctatatga accaagccac tgc                                               23


<210>  53
<211>  23
<212>  DNA
<213>  Artificial

<220>
<223>  probe

<400>  53
cgccgtcagt acttgtatag atg                                               23


<210>  54
<211>  23
<212>  DNA
<213>  Artificial

<220>
<223>  probe

<400>  54
gtcggtatcg aaaaggtact gca                                               23


<210>  55
<211>  23
<212>  DNA
<213>  Artificial

<220>
<223>  probe

<400>  55
aggcagttca acctatatct gcg                                               23


<210>  56
<211>  23
<212>  DNA
<213>  Artificial

<220>
<223>  probe

<400>  56
ggtcgtaaca ttgagaggag acg                                               23


<210>  57
<211>  23
<212>  DNA
<213>  Artificial

<220>
<223>  probe

<400>  57

ggcgatttat tgctaactgg cta                                                23

<210>  58
<211>  23
<212>  DNA
<213>  Artificial

<220>
<223>  probe

<400>  58
gcactaccgc taactatacg cta                                                23

<210>  59
<211>  23
<212>  DNA
<213>  Artificial

<220>
<223>  probe

<400>  59
ggctcgtagt actccttaca tgc                                                23

<210>  60
<211>  23
<212>  DNA
<213>  Artificial

<220>
<223>  probe

<400>  60
ggctctacaa acttgtgtcc atg                                                23

<210>  61
<211>  23
<212>  DNA
<213>  Artificial

<220>
<223>  probe

<400>  61
ggtggagtga atctcactag act                                                23

<210>  62
<211>  23
<212>  DNA
<213>  Artificial

<220>
<223>  probe

<400>  62
ctagcacaat taatcaatcc gcc                                                23

<210>  63
<211>  23
<212>  DNA
<213>  Artificial

<220>
<223>  probe

<400>  63
gcagctgaat tgctatgatc acc                                               23


<210>  64
<211>  23
<212>  DNA
<213>  Artificial

<220>
<223>  probe

<400>  64
gcctatagtg tcgattgtcc tcg                                               23


<210>  65
<211>  23
<212>  DNA
<213>  Artificial

<220>
<223>  probe

<400>  65
cgatcacgga ttaatgtcac ccc                                               23


<210>  66
<211>  23
<212>  DNA
<213>  Artificial

<220>
<223>  probe

<400>  66
aagagattta acttgagctc gcc                                               23


<210>  67
<211>  23
<212>  DNA
<213>  Artificial

<220>
<223>  probe

<400>  67
tttgttgttc gatatcaggc gtg                                               23


<210>  68
<211>  23
<212>  DNA

<213>  Artificial

<220>
<223>  probe

<400>  68
gcccgggaat agattataac gca                                              23


<210>  69
<211>  23
<212>  DNA
<213>  Artificial

<220>
<223>  probe

<400>  69
gcatttttag taatccgagc gcc                                              23


<210>  70
<211>  23
<212>  DNA
<213>  Artificial

<220>
<223>  probe

<400>  70
catggataag ttttcaagct gcg                                              23


<210>  71
<211>  23
<212>  DNA
<213>  Artificial

<220>
<223>  probe

<400>  71
gagacaggta aaccctcaga gca                                              23


<210>  72
<211>  23
<212>  DNA
<213>  Artificial

<220>
<223>  probe

<400>  72
tagcacccgt taaaacggaa atg                                              23


<210>  73
<211>  23
<212>  DNA
<213>  Artificial

<220>

<223>  probe

<400>  73
tatgtttagt tgttgaaccg gcg                                              23


<210>  74
<211>  23
<212>  DNA
<213>  Artificial

<220>
<223>  probe

<400>  74
cgatcagctc tatttccctc cca                                              23


<210>  75
<211>  23
<212>  DNA
<213>  Artificial

<220>
<223>  probe

<400>  75
agtcagttaa tcagacgtga gca                                              23


<210>  76
<211>  23
<212>  DNA
<213>  Artificial

<220>
<223>  probe

<400>  76
tggcaataca ataacgtatc gcg                                              23


<210>  77
<211>  23
<212>  DNA
<213>  Artificial

<220>
<223>  probe

<400>  77
cgcagtttgc aagaacgaac aaa                                              23


<210>  78
<211>  23
<212>  DNA
<213>  Artificial

<220>
<223>  probe

<400>  78

cgcgataatt gatacctacg ggc                                                23

<210>  79
<211>  23
<212>  DNA
<213>  Artificial

<220>
<223>  probe

<400>  79
ggggtgtgag agctttttag acg                                                23

<210>  80
<211>  23
<212>  DNA
<213>  Artificial

<220>
<223>  probe

<400>  80
gggatccgta acaagtgtgt tag                                                23

<210>  81
<211>  23
<212>  DNA
<213>  Artificial

<220>
<223>  probe

<400>  81
accactatga ttgaggaaac gcg                                                23

<210>  82
<211>  23
<212>  DNA
<213>  Artificial

<220>
<223>  probe

<400>  82
cgtctttagt atcaaccctc cgc                                                23

<210>  83
<211>  23
<212>  DNA
<213>  Artificial

<220>
<223>  probe

<400>  83
gcatacgaac ttctatatcg gcg                                                23

<210>  84
<211>  23
<212>  DNA
<213>  Artificial

<220>
<223>  probe

<400>  84
ccgtgtgtat gagtatgaca gca                                              23


<210>  85
<211>  23
<212>  DNA
<213>  Artificial

<220>
<223>  probe

<400>  85
tgctgtcttc gtgttttacc tag                                              23


<210>  86
<211>  23
<212>  DNA
<213>  Artificial

<220>
<223>  probe

<400>  86
cgatcatgta aagctaactc gcg                                              23


<210>  87
<211>  23
<212>  DNA
<213>  Artificial

<220>
<223>  probe

<400>  87
tgccgtcatt taaacgtaag ggt                                              23


<210>  88
<211>  23
<212>  DNA
<213>  Artificial

<220>
<223>  probe

<400>  88
tggcaattac agttgttaac gca                                              23


<210>  89
<211>  23
<212>  DNA

<213>  Artificial

<220>
<223>  probe

<400>  89
gagtcgaaga cctcctccta ctc                                              23


<210>  90
<211>  23
<212>  DNA
<213>  Artificial

<220>
<223>  probe

<400>  90
atgccaatat gtactcgtga ctc                                              23


<210>  91
<211>  23
<212>  DNA
<213>  Artificial

<220>
<223>  probe

<400>  91
gcatatagtg acggtaaggc gaa                                              23


<210>  92
<211>  23
<212>  DNA
<213>  Artificial

<220>
<223>  probe

<400>  92
gcctcacttg taataagcgg gac                                              23


<210>  93
<211>  23
<212>  DNA
<213>  Artificial

<220>
<223>  probe

<400>  93
gtcccaaaag cttcttacgg acg                                              23


<210>  94
<211>  23
<212>  DNA
<213>  Artificial

<220>

<223>  probe

<400>  94
ctaggtacaa caccaactgt ctc                                               23


<210>  95
<211>  23
<212>  DNA
<213>  Artificial

<220>
<223>  probe

<400>  95
tgccggttat acctttaagg acg                                               23


<210>  96
<211>  23
<212>  DNA
<213>  Artificial

<220>
<223>  probe

<400>  96
ggctggttaa atgtaaatcc gcg                                               23


<210>  97
<211>  23
<212>  DNA
<213>  Artificial

<220>
<223>  probe

<400>  97
cgcggtacta ttagaaaggg cta                                               23


<210>  98
<211>  23
<212>  DNA
<213>  Artificial

<220>
<223>  probe

<400>  98
agtcgcttaa ttactccgga tgg                                               23


<210>  99
<211>  23
<212>  DNA
<213>  Artificial

<220>
<223>  probe

<400>  99

cgctgttggt attaccttcc tcg                                              23

<210>  100
<211>  23
<212>  DNA
<213>  Artificial

<220>
<223>  probe

<400>  100
tgcagtgtaa gcaactattg tct                                              23

<210>  101
<211>  69
<212>  DNA
<213>  Artificial

<220>
<223>  primer

<220>
<221>  misc_structure
<222>  (24)..(24)
<223>  C3-linker

<220>
<221>  misc_feature
<222>  (24)..(24)
<223>  n is a, c, g, or t

<400>  101
tgttctctga ccaatgaatc tgcnaccaaa gaatatggct gaatttagta gtgttttaaa      60

taattttaa                                                              69

<210>  102
<211>  66
<212>  DNA
<213>  Artificial

<220>
<223>  primer

<220>
<221>  misc_structure
<222>  (21)..(21)
<223>  C3-linker

<220>
<221>  misc_feature
<222>  (21)..(21)
<223>  n is a, c, g, or t

<400>  102
tttttttttt tttttttttt nacctgctaa tgagatgatc ccttattttg aaaacaacta      60

ttccta                                                                 66

<210>  103
<211>  43
<212>  DNA
<213>  Artificial

<220>
<223>  probe

<400>  103
tttttttttt tttttttttt tgttctctga ccaatgaatc tgc                    43

## Description

### TECHNICAL FIELD

This application claims priority based on Japanese Patent Application No. 2012-151755 filed on July 5, 2012, the contents of which are hereby incorporated by reference into the Description. The Description relates to a nucleic acid chromatography method, a composition for nucleic acid chromatography and a kit containing the same.

### DESCRIPTION OF RELATED ART

Nucleic acid chromatography is a method for detecting target nucleic acids derived from various living organisms. In nucleic acid chromatography, a liquid containing a target nucleic acid is made to expand on a porous solid-phase carrier capable of dispersing the liquid by capillary action, causing the formation of a hybridization product with a detection probe fixed in advance on a specific region of the solid-phase carrier. The hybridization product is detected based on a signaling element that is attached to the target nucleic acid either before or during development. That is, either the signaling element or a secondary signal based on the signaling element is detected in the specific region.

### Citation List

### Patent Literature

Patent Literature 1: WO2009/034842A1
Patent Literature 2: WO2012/034842A1

### BRIEF SUMMARY OF INVENTION

In nucleic acid chromatography, a reaction time of about 10 to 15 minutes is required after the beginning of development. Moreover, the amount of the nucleic acid or the like supplied to chromatography may vary according to the nucleic acid amplification reaction or the like. Adequate detection sensitivity may not be obtained if the amount of the target nucleic acid or the like is too small.

The Description provides a nucleic acid chromatography method capable of providing stable detection, together with a composition and kit for nucleic acid chromatography.

The inventors have conducted numerous studies into more practical methods of detecting target nucleic acids, or in other words into nucleic acid chromatography methods capable of providing more stable nucleic acid detection. As a result, it was discovered that the sensitivity of detection of a target nucleic acid is improved when a water-soluble polymer such as bovine serum albumin (BSA) that plays no part in target nucleic acid detection is included in the liquid supplied to nucleic acid chromatography. The Description provides the following means based on these findings.
(1) A nucleic acid chromatography method, comprising a step of performing chromatography by supplying a chromatography liquid containing a target nucleic acid to a solid-phase carrier on which a detection probe capable of capturing the target nucleic acid is fixed, wherein
   the chromatography liquid contains one or two or more kinds of water-soluble polymers selected from the group consisting of albumin, casein, skim milk, globulin, gelatin, heparin, polyethylene glycol, polyvinylpyrrolidone, dextran, polyvinyl alcohol, carboxymethyl cellulose and polymaleimide.
(2) The method according to (1), wherein the water-soluble polymer includes albumin.
(3) The method according to (1) or (2), wherein a concentration of the water-soluble polymer in the chromatography liquid is 3.5 mass% or less.
(4) The method according to any one of (1) to (3), wherein the chromatography liquid further contains magnesium salt.
(5) The method according to (4), wherein the magnesium salt includes magnesium chloride.
(6) The method according to (4) or (5), wherein a concentration of the magnesium salt is 0.5 mM or more and 10.0 mM or less.
(7) The method according to any one of (1) to (6), wherein the chromatography liquid further contains a surfactant.
(8) The method according to any of (1) to (7), wherein the chromatography liquid further contains a label for distinguishing the target nucleic acid.
(9) The method according to (8), wherein the label is colored particles.
(10) The method according to any one of (1) to (9), further comprising a step of preparing the chromatography liquid prior to the chromatography step, the chromatography liquid including a nucleic acid amplification reaction solution containing the target nucleic acid as an amplification product.
(11) The method according to any one of (4) to (6), wherein at least a part of the magnesium salts is replenished by magnesium salt in the nucleic acid amplification reaction solution.
(12) A composition for nucleic acid chromatography, the composition containing one or two or more kinds of water-soluble polymers selected from the group consisting of albumin, casein, skim milk, globulin, gelatin, heparin, polyethylene glycol, polyvinylpyrrolidone, dextran, polyvinyl alcohol, carboxymethyl cellulose and polymaleimide.
(13) The composition according to (12), wherein the water-soluble polymer includes albumin.
(14) The composition according to (12) or (13), containing the water-soluble polymer so that a final concentration of the water-soluble polymer is 3.5 mass% or less.
(15) The composition according to any one of (12) to (14), further containing a magnesium salt.
(16) The composition according to (15), wherein the magnesium salt includes magnesium chloride.
(17) The composition according to any of (12) to (16), containing the magnesium salt so that a final concentration of the magnesium salt is 0.5 mM or more and 10.0 mM or less.
(18) The composition according to any of (12) to (17), further containing a surfactant.
(19) The composition according to any of (12) to (18), he composition being configured to be added to a nucleic acid amplification reaction solution containing an amplification product corresponding to a target nucleic acid.
(20) A kit for nucleic acid chromatography, the kit comprising a composition for nucleic acid chromatography containing one or two or more kinds of water-soluble polymers selected from the group consisting of albumin, casein, skim milk, globulin, gelatin, heparin, polyethylene glycol, polyvinyl pyrrolidone, dextran, polyvinyl alcohol, carboxymethyl cellulose and polymaleimide.
(21) The kit according to (20), wherein the composition for nucleic acid chromatography also contains a magnesium salt.

### BRIEF DESCRIPTION OF DRAWINGS

FIG. 1A shows an outline of one example of an amplification step in the detection method of the invention;
FIG. 1B shows an outline of one example of another amplification step in the detection method of the invention;
FIG. 2A shows one example of the flow of the detection method of the invention;
FIG. 2B shows another example of the flow of the detection method of the invention;
FIG. 2C shows another example of the flow of the detection method of the invention;
FIG. 3 shows a chromatography body used in an example;
FIG. 4 shows nucleic acid chromatography performed using the chromatography body shown in FIG. 3;
FIG. 5 shows the results of nucleic acid chromatography in Example 1;
FIG. 6 shows the results of nucleic acid chromatography in Example 2;
FIG. 7 shows the results of nucleic acid chromatography in Example 3;
FIG. 8 shows the results of nucleic acid chromatography in Example 4;
FIG. 9 shows the results of nucleic acid chromatography in Example 5; and
FIG. 10 shows the results of nucleic acid chromatography in Example 6.

### DETAILED DESCRIPTION OF INVENTION

The Description provides a nucleic acid chromatography method, a composition for nucleic acid chromatography and a kit for nucleic acid chromatography. In the nucleic acid chromatography method disclosed in the Description, one or two or more kinds of specific water-soluble polymers are included in a nucleic acid chromatography liquid containing a target nucleic acid. The composition disclosed in the Description is a composition for preparing a nucleic acid chromatography liquid, and contains one or two or more kinds of specific water-soluble polymers. The kit disclosed in the Description is provided with this composition. The nucleic acid chromatography method, composition and kit disclosed in the Description are capable of detecting target nucleic acids with good detection sensitivity because one or two or more kinds of specific water-soluble polymers are included in the liquid supplied to nucleic acid chromatography. Thus, for example a target nucleic acid can be detected stably even if there are fluctuations in the quantity of the target nucleic acid for example. Because detection sensitivity is good, moreover, a target nucleic acid can be detected rapidly. The reason for this is not necessarily clear, but it may be attributable to an increased concentration of the target nucleic acid in the probe region.

In the Description, a target nucleic acid is any nucleic acid the existence and/or amount of which is to be detected, without any particular limitations. The target nucleic acid may be natural or artificially synthesized. Natural target nucleic acids include bases or base sequences that are genetic markers for physical properties and genetic diseases, and for the occurrence, diagnosis, prognosis, and drug and treatment selection for cancer and other specific disorders in humans, non-human animals and other organisms. Typical examples include SNPs and other polymorphisms and congenital and acquired mutations. Nucleotide sequences derived from pathogenic bacteria, viruses and other microorganisms are also examples of nucleotide sequences of target nucleic acids. A synthetic target nucleic acid may be a nucleic acid that has been synthesized artificially for some identification purpose. Other examples include amplification products obtained by performing a nucleic acid amplification reaction on some kind of natural or artificial nucleic acid.

In the Description, the source of the target nucleic acid is not particularly limited, but examples of samples that may contain target nucleic acids include various samples from living organisms (blood, urine, sputum, tissue, cells (including cultured animal cells from various animals, cultured plant cells, and cultured microbial cells)), and DNA extract samples of DNA extracted from such biological samples and the like.

In the Description, a "nucleic acid" is a polymer made up of nucleotides, the number of which is not limited. Hence, target nucleic acids encompass oligonucleotides formed by linking tens of nucleotides, as well as longer polynucleotides. In addition to single-stranded or double-stranded DNA, target nucleic acids include single-stranded or double-stranded RNA, as well as DNA/RNA hybrids, DNA/RNA chimeras and the like. Nucleic acids may consist of natural bases, nucleotides and nucleosides, but may also consist partly of non-natural bases, nucleotides and nucleosides. In addition to all kind of DNA and RNA including cDNA, genome DNA, synthetic DNA, mRNA, total RNA, hnRNA and synthetic RNA, nucleic acids include peptide nucleic acids, morpholino nucleic acids methylphosphonate nucleic acids, S-oligonucleic acids and other artificially synthesized nucleic acids. These may be single-stranded or double-stranded.

In the case of a double strand, a partially double-stranded structure having a single-stranded part at one or both ends is also possible. With a partially double-stranded structure, the heat-denaturing step required with a complete double strand can be omitted because hybridization with the detection probe is possible at the single-stranded part. Such a partially double-stranded structure may be for example a structure in which one end (the 5' end or 3' end) of one strand of the double strand protrudes from the double-stranded part as a single strand, and one end (the 3' end or 5' end) of the other strand also protrudes in the same way as a single strand. It may also be a structure having one strand that protrudes from the paired strand at both ends (the 5' end and 3' end) of one strand of the double strand.

Examples of such partially double-stranded structures include amplification products of nucleic acid amplification reactions as described in WO 2012/034842, WO 2006/095550, Japanese Patent Application Publication No. H5-252998 and the like. These amplification products are, respectively, a double-stranded DNA having a partially double-stranded structure with protruding ends consisting of natural DNA at the 5' ends of both strands, a double-stranded DNA having a partially double-stranded structure with a protruding end consisting of a natural DNA aptamer at the 5' end of one strand, and a partially double-stranded structure with a protruding end consisting of non-natural DNA at the 5' end of one strand.

This type of double-stranded DNA can be obtained by performing a nucleic acid amplification reaction on a sample containing a target nucleic acid, using a primer set comprising a first primer having at the 5' end a first tag strand corresponding to a single-stranded part of a first strand, joined via a linking site that inhibits a DNA polymerase reaction to a first recognition sequence capable of hybridizing with a target nucleic acid, and a second primer having at the 5' end a second tag strand corresponding to a single-stranded part of a second strand paired with the first strand, joined via a linking site that inhibits or arrests a DNA polymerase reaction to a first recognition sequence capable of hybridizing with the target nucleic acid. Nucleic acid amplification using primers containing such linking sites is discussed below.

Typical and non-limiting specific examples of the disclosures of the Description are explained in detail below with reference to the drawings. These detailed explanations are aimed simply at showing preferred examples of the disclosures of the Description in detail so that they can be implemented by a person skilled in the art, and are not intended to limit the scope of the disclosures of the Description. The additional features and disclosures disclosed below may be used separately or together with other features and inventions to provide a further improved method of detecting a target nucleic acid or the like.

The combinations of features and steps disclosed in the detailed explanations below are not essential for implementing the disclosures of the Description in the broadest sense, and are presented only for purposes of explaining typical examples of the disclosures of the Description in particular. Moreover, the various features of the typical examples above and below and the various features described in the independent and dependent claims do not have to be combined in the same way as in the specific examples described here, or in the listed order, when providing addition useful embodiments of the disclosures of the Description.

All features described in the Description and/or Claims are intended as individual and independent disclosures restricting the initial disclosures and the claimed matter specifying the invention, separately from the constitution of features described in the Examples and/or Claims. Moreover, all descriptions of numerical ranges and groups or sets are intended to include intermediate configurations for purposes of restricting the initial disclosures and the claimed matter specifying the invention.

Embodiments of the present invention are explained below.

### (Nucleic acid chromatography method)

The present nucleic acid chromatography method (hereunder simply called the chromatography method) comprises a step of performing chromatography by supplying a nucleic acid chromatography liquid containing a target nucleic acid (hereunder simply called a chromatography liquid) to a solid-phase carrier on which is fixed a detection probe capable of capturing the target nucleic acid. The chromatography body and chromatography liquid for performing nucleic acid chromatography are explained below, after which the chromatography process is explained.

### (Chromatography body)

The chromatography body comprises a solid-phase carrier and one or two or more kinds of detection probes fixed on the solid-phase carrier. A conventionally known carrier may be adopted for the solid-phase carrier, without any particular limitations. For example, a so-called porous material consisting primarily of a polymer such as polyethersulfone, nitrocellulose, nylon, vinylidene polyfluoride or the like may be used for the solid-phase carrier. Filter paper or another cellulose material may also be used favorably. The form of the chromatography body is not particularly limited. It may be in the form of a sheet, thin bar, or other form that allows expansion and dispersal of the chromatography liquid by capillary action.

Each detection probe contains a detection sequence capable of hybridizing with at least part of a target nucleic acid so as to capture the target nucleic acid. The detection sequence is preferably complementary to the aforementioned part of the target nucleic acid. The detection sequence of the detection probe has a nucleotide sequence that is sufficiently complementary to allow specific hybridization with at least part of the target nucleic acid. Preferably, it has a nucleotide sequence that is entirely complementary.

Such a detection sequence can be designed to be complementary to a characteristic sequence of the target nucleic acid, but it can also be designed independently of the target sequence. Designing the sequence independently of the target sequence makes it possible to inhibit or eliminate non-specific binding between the detection sequences of multiple detection probes, and to incorporate considerations of optimum hybridization temperature, time and other hybridization conditions into the design. It also makes it possible to always use the same detection probe independently of the kind of target nucleic acid. In this case, a nucleotide sequence capable of hybridizing with such a detection probe is included in the target nucleic acid. Typically, primers for PCR and other nucleic acid amplification reactions are designed to include such nucleotide sequences.

The preferred length of the detection sequence is not specifically limited but may be preferably, for example, 20 bases to 50 bases. The detection sequence having the bases in length can ensure, in general, both of the specificity of each of the detection sequence and hybridization efficiency. For example, the detection sequence having the bases in length may be selected from 46 bases sequence which base sequences described by SEQ IDs: 1 to 100 and their complementary sequences are combined and their modified sequences which addition or deletion of base is made. More preferably, the length of the detection sequence is 20 bases to 25 bases. For example, the detection sequence having such number of bases in length may be selected from sequences of 23 bases, each of which is described by any one of SEQ IDs: 1 to 100 and their complementary sequences and their modified sequences which addition or deletion of base is made arbitrarily. Tag sequence of the first primer is the sequence which pairs the detection sequence. Therefore, the length of the tag sequence is preferably 20 to 50 bases, more preferably 20 to 25 bases as same as the detection sequence.

One or two or more kinds of detection probes may be fixed on a single solid-phase carrier. The form of fixing is not particularly limited. A known fixing method may be used. For example, fixing can be accomplished by static interaction between the detection probe and the surface of the solid-phase single carrier, or by covalent binding between functional groups in the detection probes and functional groups in the material of the solid-phase carrier (either pre-existing functional groups or functional groups added for fixing purposes).

The regions of the solid-phase carrier on which the detection probes are fixed (probe regions) may be formed with any pattern. This may be a pattern of dots in any configuration, or a pattern of streaks, or any other pattern. Multiple probe regions are preferably provided at fixed intervals in the direction of expansion in the form of streaks perpendicular to the expansion direction of the chromatography liquid. Preferably one probe region corresponds to one kind of detection probe.

The chromatography body may also be provided with additional known sites for example. A supply part to which the chromatography liquid is supplied may be provided upstream of the probe region. A complexing part for complexing a label with the supplied target nucleic acid may also be provided upstream of the probe region, and at the same location as or downstream of the supply part. A detection window or colored marker may also be provided showing the probe region so that this region can be detected visually with ease. Furthermore, an intake part for collecting the liquid after completion of development may be provided downstream from the probe region.

In addition to the probe region with the detection probes fixed thereon, the solid-phase carrier may be provided with a marker site or the like for indicating that the chromatography liquid has been sufficiently expanded. Like the probe regions, these sites are all formed from porous materials to allow expansion of the chromatography liquid, and are composed so as not to inhibit continuous expansion of the chromatography liquid to all sites. For example, multiple such sites are provided on one solid-phase carrier. The label is discussed below.

### (Chromatography liquid)

The chromatography liquid is a liquid that expands and moves by capillary action through the solid-phase carrier of the chromatography body, and is the medium that moves the target nucleic acid to the solid phase carrier. In the Description, "chromatography liquid" means a liquid of a composition and condition for application to the chromatography body. The chromatography composition described below is a composition for preparing such a chromatography liquid.

The liquid component of the chromatography liquid disclosed in the Description is an aqueous medium. The aqueous medium is not particularly limited, but examples include, water, organic solvents compatible with water, and mixtures of water with one or two or more kinds of organic solvents. Organic solvents that are compatible with water are well known to those skilled in the art, and include lower alcohols with about 1 to 4 carbon atoms, DMSO, DMF, methyl acetate, ethyl acetate and other esters, and acetone and the like.

The chromatography liquid may include a component for adjusting the pH of the liquid to a definite range. This is done to ensure a pH range that provides a suitable dissociation state, stability and expansion environment for the target nucleic acid. The buffer salts depend on the target pH value, which is normally in the range of 6.0 to 8.0, or preferably 7.0 to 8.0. The components for obtaining such a pH value include acetic acid and sodium acetate (acetate buffer), citric acid and sodium citrate (citrate buffer), phosphoric acid and sodium phosphate (phosphate buffer) and the like for example. Other examples include phosphate buffered saline (PBS).

The chromatography liquid contains a water-soluble polymer. A water-soluble polymer here means a water-soluble polymer other than the target nucleic acid that does not impede specific hybridization between the target nucleic acid and the detection probe. Such a water-soluble polymer is a natural or synthetic water-soluble polymer that is either heterogenous or has a nucleotide sequence unrelated to the target nucleic acid or detection probe. By including the water-soluble polymer in the chromatography liquid, it is possible to concentrate the target nucleic acid in the probe region and increase and stabilize the detection sensitivity of the target nucleic acid. Rapidity of detection is also promoted.

Examples of such water-soluble polymers include albumin, casein, skim milk, globulin, gelatin (mammalian gelatin from cows or pigs or fish gelatin), heparin, polyethylene glycol, polyvinylpyrrolidone, dextran, polyvinyl alcohol, carboxymethyl cellulose and polymaleimide. This is because these water-soluble polymers are normally heterogenous with the target nucleic acid and detection probe. Any of these may be used individually, or two or more may be combined.

Of the water-soluble polymers, albumin, casein and skim milk are preferred, and albumin is especially desirable. Serum albumin from one or two or more kinds of animals selected from the group consisting of cows, pigs, goats, guinea pigs, rabbits, humans, dogs, rats, cats, mice, monkeys, donkeys, hamsters, horses, chickens and turkeys can be used as the albumin. Preferably this includes bovine serum albumin. The serum itself may also be used instead of the albumin. These kinds of water-soluble polymers are all available commercially.

A water-soluble polymer other than the aforementioned proteins and polymers may also be used. This may be DNA and RNA. Examples of such water-soluble polymers include DNA, RNA and other polymers. Typical examples include fish DNA, such as herring sperm DNA and other herring DNA and salmon sperm DNA and other salmon DNA. Artificial examples include synthetic DNA and RNA consisting of nucleotide sequences unrelated to the target nucleic acid or detection probe. Examples include poly(A), poly(G), poly(T), poly(C), poly(dA), poly(dG), poly(dT), poly(dC), poly(dU) and other homopoly-ribonucleotide polymers and polymer-polydeoxyribonucleotides, and block copolymers comprising blocks of these and the like.

The concentration of the water-soluble polymer in the chromatography liquid is not particularly limited within the range at which detection of the target nucleic acid is stabilized, but is 3.5 mass% or less because above 3.5 mass%, the improvement in the stability of detection sensitivity is not proportional to the increase in the content of the water-soluble polymer. 3 mass% or less is more preferred, and 2.5 mass% or less is still more preferred. 2 mass% or less is also allowable. The lower limit of the concentration of the water-soluble polymer is preferably 0.1 mass%. This is because below 0.1 mass%, it is hard to obtain any effect from including the water-soluble polymer. The lower limit is more preferably 0.3 mass%, or still more preferably 0.4 mass%, or yet more preferably 0.5 mass%.

The chromatography liquid may also contain a magnesium salt. By including magnesium salt in addition to the water-soluble polymer, it is possible to further concentrate the target nucleic acid in the probe region and thereby improve the stability, sensitivity and rapidity of target nucleic acid detection. This effect is a peculiar effect of magnesium salts, and is obtained hardly at all with potassium salts or sodium salts.

The magnesium salt is an ionic compound containing magnesium in cationic form. Typical examples include magnesium hydroxide, magnesium chloride, magnesium bromide, magnesium iodide and other inorganic salts, and magnesium acetate, magnesium citrate, magnesium malate and other organic acid salts and the like. The magnesium salt is not particularly limited, but one or a combination of two or more may be used. Preferably magnesium chloride is included.

The concentration of the magnesium salt is determined appropriately within the range at which the stability of detection of the target nucleic acid can be improved. A range of 0.5 mM to 10.0 mM is preferred because synergy with the water-soluble polymer is hard to achieve below 0.5 mM, while non-specific hybridization occurs above 10.0 mM. 1mM or more is more preferred, 2 mM or more is still more preferred, and 3 mM or more is yet more preferred. Even at about 8.0 mM, specific hybridization between the target nucleic acid and the detection probe is not inhibited, and it is possible to improve the detection sensitivity of the target nucleic acid and achieve rapid detection. 5 mM or less is also possible.

The chromatography liquid may also include a metal salt other than a magnesium salt. This is not particularly limited, but for example a potassium salt or sodium salt may be included.

The chromatography liquid may also contain a surfactant. By including a surfactant, it is possible to reduce boundary tension at the air-liquid boundary and solid-liquid boundary, and improve the wetting properties. Improving the wetting properties has the effect of increasing the expansion speed of the chromatography liquid, allowing more rapid detection. The surfactant is not particularly limited, but examples include polyoxyethylene octylphenyl ether, polyoxyethylene sorbitan monolaurate, polyoxyethylene alkylallyl ether phosphate ester, polyoxyethylene alkyl ether phosphate ester, polyoxyethylene alkylphenyl ether, sodium dodecylsulfate (SDS) and the like. More specific examples include Triton X-100 (consisting primarily of polyethylene glycol mono-p-isooctylphenyl ether), Tween 20 (consisting primarily of polyoxyethylene sorbitan monolaurate) and Tween 80 (consisting primarily of polyoxyethylene sorbitan monolaurate).

The concentration of the surfactant is not particularly limited, but 0.01 mass% to 1.0 mass% is preferred. 0.05 mass% to 0.5 mass% is more preferred, 0.05 mass% to 0.35 mass% is still more preferred, 0.2 mass% is yet more preferred, and 0.1 mass% or less is most preferred.

The chromatography liquid contains a target nucleic acid. The form of the target nucleic acid is not particularly limited as discussed above. It may be in the form of an amplification product from a known PCR or other nucleic acid amplification reaction. That is, a nucleic acid amplification step may be provided prior to the chromatograph step described below.

When the target nucleic acid is in single-stranded form, a heat-denaturing step (heating between about 90°C and 95°C) is not required to dissociate the double strand in the chromatography liquid. Heat denaturing may also be omitted when the target nucleic acid is a partial double strand having a single strand that hybridizes with the detection probe. When the target nucleic acid is a double strand such as an amplification product of a nucleic acid amplification reaction, on the other hand, it must be converted to a single strand to allow hybridization with the detection probe. That is, the chromatography liquid containing the target nucleic acid must be subjected to a step of heating to heat denature the double strand prior to the chromatography step.

When the target nucleic acid is obtained as an amplification product of a nucleic acid amplification reaction prior to the chromatography step, there may be a step of preparing a chromatography liquid comprising a nucleic acid amplification reaction solution containing an amplification product. That is, using the nucleic acid amplification reaction solution as part of the chromatography liquid, both BSA and a magnesium salt can be added to obtain the chromatography liquid. This can then be supplied easily to the chromatography step without isolating the DNA from the nucleic acid amplification reaction solution. Because this chromatography liquid contains a water-soluble polymer and a magnesium salt, a target nucleic acid can be detected stably even using the nucleic acid amplification reaction solution as part of the chromatography liquid.

One example of a nucleic acid amplification step for obtaining an amplification product with a partially double-stranded structure as discussed above is explained here.

### (Amplification step)

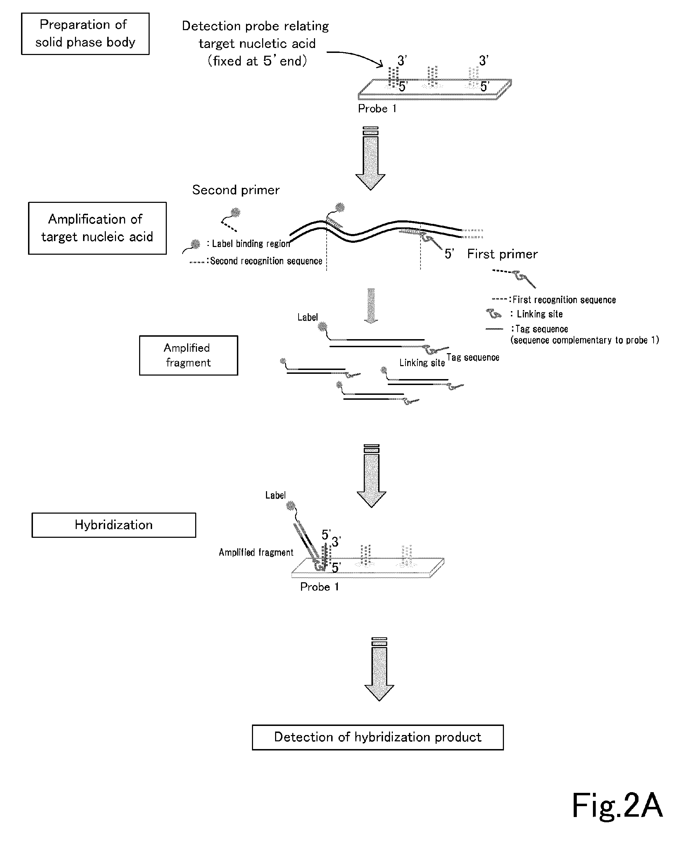

As shown in FIG. 2A, the amplification step is performed using a first primer and a second primer. Various known methods for obtaining double-stranded DNA fragments by amplifying DNA by a DNA polymerase reaction such as PCR can be used as the nucleic acid amplification method in the nucleic acid amplification step.

### (First primer)

The first primer comprises a tag sequence complementary to the detection probe associated with the target nucleic acid, and a first recognition sequence that recognizes a first base sequence in the target nucleic acid. The lengths of these base sequences are not particularly limited, and can be determined appropriately according to the nature of the target sequence of the target nucleic acid.

### (First recognition sequence)

The first recognition sequence is a sequence for amplifying the target nucleic acid by nucleic acid amplification, and can hybridize specifically with a first base sequence constituting part of the target sequence of the target nucleic acid. The first recognition sequence is designed with a degree of complementarity that allows it to hybridize very selectively with the first base sequence. Preferably it is designed to be entirely complementary (specific).

### (Tag sequence)

A tag sequence is a sequence that makes it possible for the amplified fragment to hybridize with the detection probe, and detect the target nucleic acid. Hence, a tag sequence is designed so that it can hybridize with the detection sequence of the detection probe for each target nucleic acid. Typically, it is a base sequence complementary to the detection sequence. Therefore, one target nucleic acid is matched to one detection probe. As explained above, the base length of the tag sequence preferably matches the base length of the detection sequence of the detection probe, and is preferably 20 to 50 bases or more preferably 20 to 25 bases.

The first base sequence and second base sequence in the target nucleic acid may be configured in any way relative to the target nucleic acid. For example, when detecting a mutation on DNA, they may be made to contain mutation sites in one or two or more bases only in one base sequence, or may contain mutation sites in both base sequences. The first primer has such a tag sequence and first recognition sequence, and together with the natural bases or artificial homologs of natural bases that constitute these base sequences, it also has a backbone that allows base pairing with a natural nucleic acid. Typically, it is an oligonucleotide or derivative thereof.

### (Linking site)

The part of the primer having the tag sequence is not directly linked to the other part of the primer having the first recognition site, and there is a linking site between the two. When incorporated into the template strand, the linking site is a site capable of inhibiting or arresting a DNA polymerase reaction. The DNA polymerase reaction does not further elongate the DNA strand when there is no nucleic acid (or bases) to serve as a template. Therefore, the linking site of the invention has a structure that cannot serve as a template during DNA elongation by DNA polymerase. That is, this linking site does not contain natural bases or natural base derivatives (natural bases or the like) that pair with natural bases. By not including such natural bases and the like, it is possible to prevent the site from being a template, and inhibit or prevent DNA strands from being elongated by DNA polymerase. Therefore, this linking site may consist solely of a simple skeletal strand having no natural bases or the like. That is, it may be a sugar-phosphate backbone or other known backbone used in artificial oligonucleotides. The DNA polymerase includes various known DNA polymerases. Typical examples include DNA polymerases used in various kinds of PCR and other nucleic acid amplification methods.

This linking site may also be a chain-like linking group containing a single-stranded structure with an element number of 2 to 40, adjoining a nucleotide via a phosphate diester bond. If the element number is 1 or less, the DNA polymerase reaction may not be completely inhibited or arrested, while if the element number is over 40, nucleotide solubility may be diminished. Considering the effect of inhibiting or arresting the DNA polymerase reaction, the element number of the chain-like linking group is preferably 2 to 36 or more preferably 3 to 16.

This linking site contains a single bond in order to facilitate rotation at the linking site, and examples of single bonds include carbon-carbon, carbon-oxygen, carbon-nitrogen and S-S single bonds. This linking site preferably consists primarily of this single bond. As long as the linking site contains a single bond an aromatic ring or cycloalkane may also be included in part of the site.

An optionally substituted alkylene chain or polyoxyalkylene chain with an element number of 2 to 40 is preferably included as this linking site. Such a chain-like linking structure is structurally simple and easy to introduce as a linking site.

One example of this linking site is the linking site represented by Formula (1) below:

5'-O-CₘH₂ₘ-O-3' Formula (1)

(wherein 5' represents the oxygen atom of a phosphate diester bond at the 5' end, 3' represents the phosphorus atom of a phosphate diester bond at the 3' end, and m is an integer from 2 to 40).

In Formula (1), m is more preferably 2 to 36, or still more preferably 3 to 16. A substituent of H in Formula (1) is typically an alkyl group, alkoxy group, hydroxyl group or the like. The number of carbon atoms in an alkyl group or alkoxy group is preferably 1 to 8, or more preferably 1 to 4. When there are 2 or more substituents, they may be the same or different. Preferably there are no substituents.

Another example of a linking site is the linking site represented by Formula (2) below:

5'-(OCₙH₂ₙ)₁-O-3' Formula (2)

(wherein 5' represents the oxygen atom of a phosphate diester bond at the 5' end, 3' represents the phosphorus atom of a phosphate diester bond at the 3' end, n is an integer from 2 to 4,1 is 2 or an integer greater than 2, and (n + 1) x 1 is 40 or an integer smaller than 40).

In Formula (2), (n + 1) x 1 is preferably 2 to 36, or more preferably 3 to 16. A substituent of H in Formula (2) may be similar to the substituent in Formula (1).

The chain-like site shown below is an example of this linking site.

[C1]

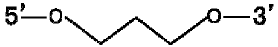

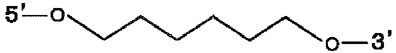

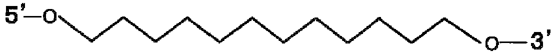

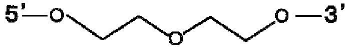

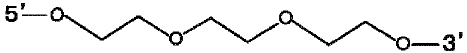

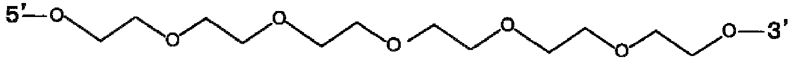

[C2]

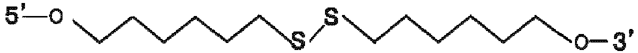

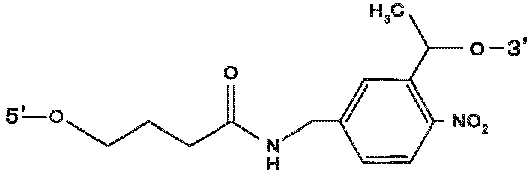

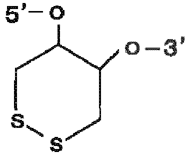

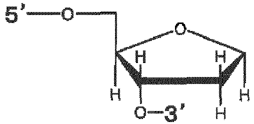

Moreover, other examples of this linking site include nucleic acid sequences with three-dimensional structures such as strong hairpin structures or pseudoknot structures that inhibit the progress of the polymerase, L-type nucleic acids, artificial nucleic acids and other target nucleic acid natural nucleic acids, and non-nucleic acid structures such as RNA and lipid chains. Examples of artificial nucleic acids include peptide nucleic acids, crosslinked nucleic acids, azobenzene and the like.

The first primer has a first recognition sequence and a tag sequence, and apart from the natural bases or artificial homologs of natural bases that constitute these base sequences, it consists principally of a backbone that allows base pairing with a natural nucleic acid. Typically it is an oligonucleotide or derivative thereof.

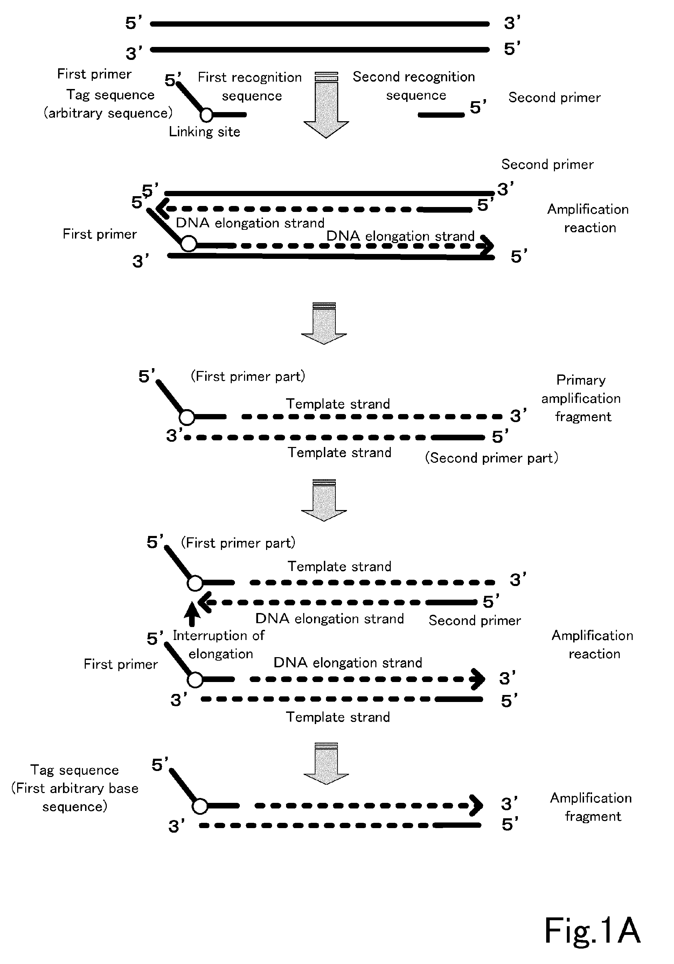

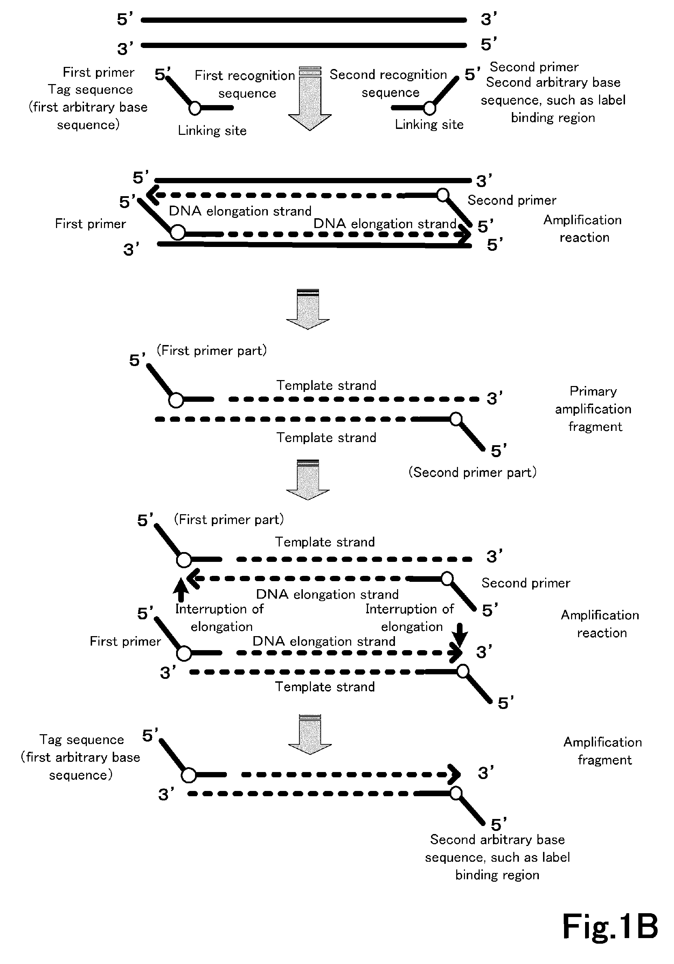

The first primer preferably has a tag sequence, a linking site and a first recognition sequence in that order from the 5' end. With such a configuration, when a DNA strand amplified by such a primer becomes a template strand for amplification, the elongation reaction is inhibited or arrested on the 5' side of the linking site derived from the first primer in the template strand, or in other words further towards the 3' end in the DNA strand elongated with DNA polymerase. This results in an amplified fragment having as its 3' end a base that pairs with a base (or a base near a base) of a nucleotide adjacent to the 3' side of the linking site derived from the first primer in the template strand, or with a base adjacent to that base, and lacking a complement strand to the tag sequence in the first primer (FIGS. 1A and 1B, FIGS. 2A to 2C).

A sequence unrelated to the tag sequence or first recognition sequence may also be included near the linking site, or in other words on the 3' and 5' side of the linking site. This makes it possible to reduce or avoid the effect of unintentionally promoting or arresting the DNA elongation reaction with respect to the tag sequence or first recognition sequence in the elongated strand due to the presence of the linking site when the first primer becomes a template strand.

### (Second primer)

As shown in FIG. 2A, the second primer contains a second recognition sequence that recognizes a second base sequence in the target nucleic acid. The lengths of these base sequences are not particularly limited, and may be determined appropriately according to the nature of the target sequence in the target nucleic acid.

### (Second recognition sequence)

The second recognition sequence is a sequence for amplifying a target nucleic acid with a first primer by nucleic acid amplification, and is capable of hybridizing specifically with a second base sequence constituting another part of the target sequence in the target nucleic acid. The second recognition sequence is designed to be sufficiently complementary so that it can hybridize very selectively with the second base sequence. Preferably it is designed to be completely complementary (specific).

### (Label-binding region)

As shown in FIG. 2A, the label-binding region may be provided in advance with a label. The purpose of the label is to detect a double-stranded DNA fragment bound to the detection probe on the solid phase body. A conventionally known substance may be selected and used appropriately as the label. Possibilities include various dyes including fluorescent substances that emit a fluorescent signal by themselves when excited, as well as substances that emit various signals in combination with second components by enzyme reactions or antigen-antibody reactions. Typically, a fluorescent label such as Cy3, Alexa555, Cy5 or Alexa647 may be used. Other possibilities include a combination of biotin and streptavidin HRP, and detection using coloration by treatment with a substrate using DIG or the like. The label-binding region is provided with the label linked to the second base sequence by a known method, either directly or via a suitable linker.

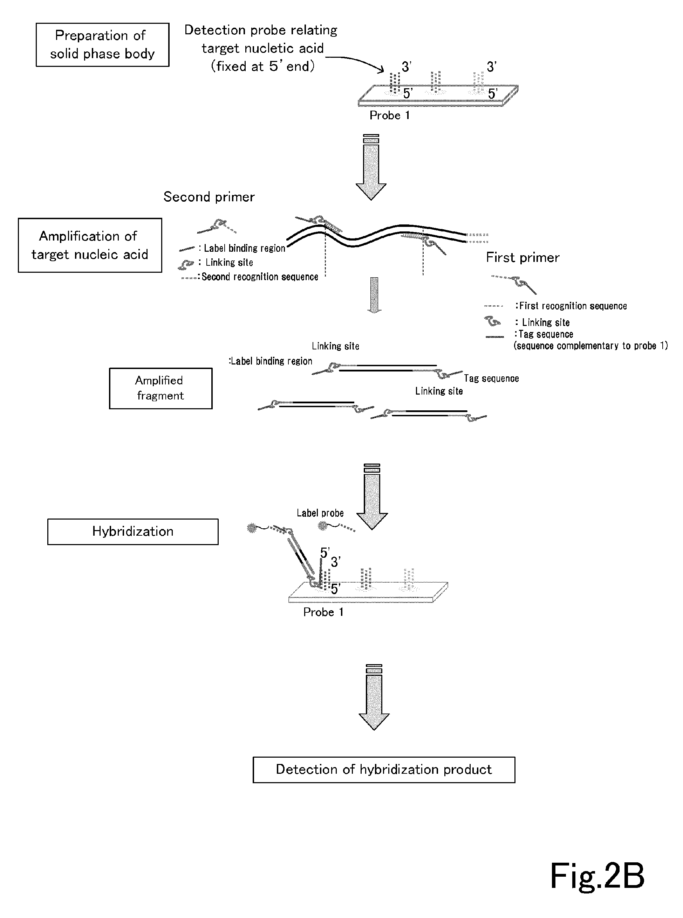

As shown in FIG. 2B, the second primer may be configured so that the label-binding region can bind the label. That is, it may be capable of binding a labeling probe comprising a specific base sequence, a label, and a base sequence that recognizes a label binding sequence. Such a labeling probe is supplied to a double-stranded DNA fragment that has hybridized with a detection probe on the solid-phase body in the hybridization step or detection step (discussed below), and can label the fragment.

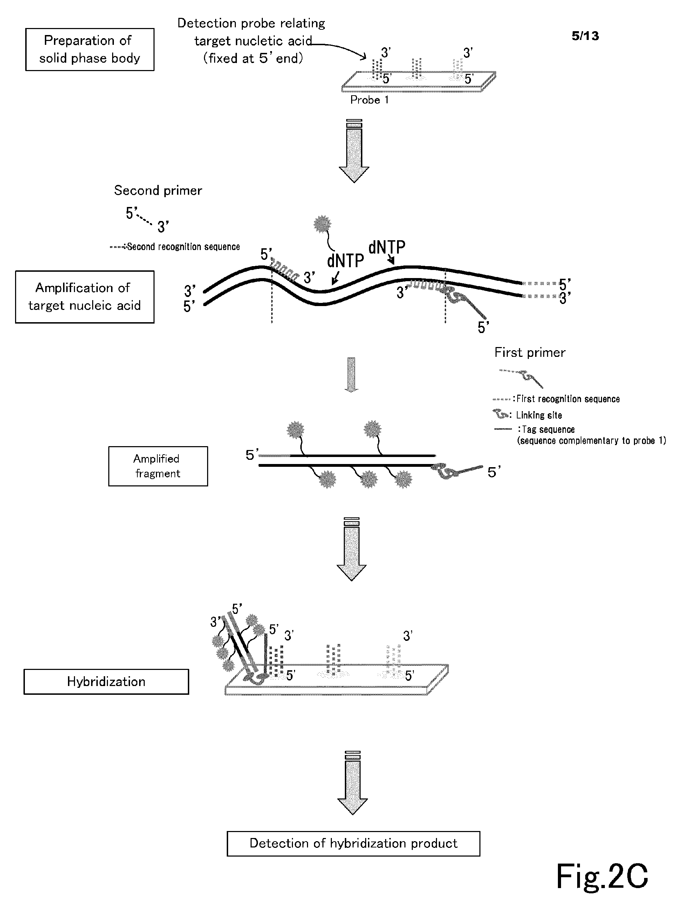

As shown in FIG. 2C, the second primer may not be provided with a label-binding region. That is, a labeled amplified fragment comprising a label introduced into a DNA elongation site of an amplified fragment can be obtained by performing nucleic acid amplification using nucleoside triphosphates including a nucleoside derivative triphosphate provided with a label in the amplification step.

The second primer has a label-binding region as necessary in addition to the second recognition sequence, and has natural nucleotides or artificial bases homologous to these making up the nucleotide sequence of the second recognition sequence, as well as a backbone that allows base pairing with natural nucleic acids. Typically, it is an oligonucleotide or derivative thereof.

### (Linking site)

When there is a label-binding region, the label-binding region and the second recognition sequence may be linked directly, or there may be a linking site between the two. As shown in FIG. 2B, this is desirable when the label-binding region has a base sequence that interacts with and binds the labeling probe. The linking site has been explained already with reference to the first primer.

In the second primer, the label-binding region, linking site and second recognition sequence are preferably arranged in that order from the 5' end. With this configuration, when a DNA strand amplified by the second primer becomes a template strand for amplification by the first primer, the elongation reaction is arrested or inhibited further towards the 5' end from the linking site derived from the second primer in the template strand, or in other words further toward the 3' end in the new DNA strand elongated by the DNA polymerase. This results in a DNA amplified fragment having as its 5' end a base that pairs with a base (or a base near a base) of a nucleotide adjacent to the 3' end of the linking site derived from the second primer in the template strand, and lacking a complement strand to the (base sequence of) the label-binding region in the second primer (FIG. 1B, FIG. 2B).

A sequence unrelated to the label-binding region or second recognition sequence may also be included near the linking site, or in other words on the 3' side and 5' side of the linking site. This makes it possible to reduce or avoid the effect of unintentionally promoting or arresting the DNA elongation reaction with respect to the label-binding region or second recognition sequence in the elongated strand due to the presence of the linking site when the second primer becomes a template strand.

Such a primer can be synthesized in accordance with ordinary oligonucleotide synthesis methods. For example, the linking site may be synthesized using a phosphoramidite reagent having an alkylene chain. Such reagents are well known, and can be obtained from GlenResearch or the like for example. An example is the following reagent. In the formula below, DMT represents a typical dimethoxytrityl group as a hydroxyl-protecting group, but this may also be another known hydroxyl-protecting group. PA in the formula below represents a phosphoramidite group.

[C3]

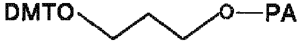

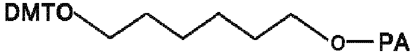

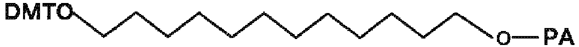

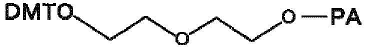

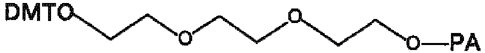

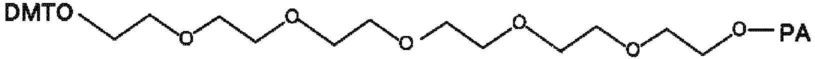

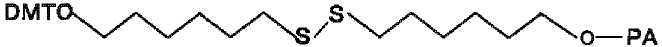

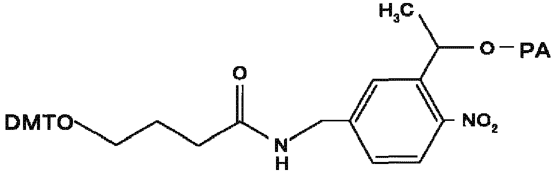

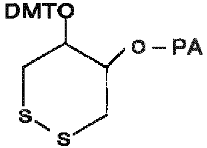

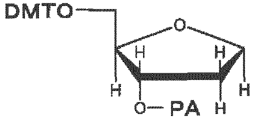

Nucleic acid amplification is performed using these primers. Various known methods may be applied to nucleic acid amplification as explained above, but typically PCR as represented by multiplex PCR are employed. When performing the nucleic acid amplification step, the solution composition, temperature control and the like can be set appropriately by a person skilled in the art.

As explained above, when a first primer having a tag sequence, a linking site and a first recognition sequence in that order from the 5' end and a second primer having a label-binding region, a linking site and a second recognition sequence in that order from the 5' end are used to perform PCR on a sample that may contain a target nucleic acid, template strands derived from the first and second primers and containing those primers are formed by a DNA elongation reaction with a DNA polymerase as shown in (a) to (c) of FIG. 1B.

A DNA elongation reaction with a DNA polymerase is then performed again using a second primer and first primer different from the primers from which the template strands were derived. As shown in (d) and (e) of FIG. 1B, in a DNA elongation reaction using a DNA polymerase on template strands beginning with the second primer and including the first primer, DNA elongation is inhibited or arrested on the 5' side of the linking site derived from the first primer in the template strands, or in other words towards the 3' end from the linking site in the elongated strand.

As shown in (d) and (e) of FIG. 1B, moreover, in a DNA elongation reaction using a DNA polymerase on template strands beginning with the first primer and including the second primer, DNA elongation is inhibited or arrested on the 5' side of the linking site derived from the second primer in the template strands, or in other words towards the 3' end from the linking site in the elongated strand.

As shown in FIG. 2B, the resulting amplified fragment is a double-stranded DNA fragment having a single-stranded tag sequence and label-binding region protruding at either 5' end, and having a double-stranded part that includes the first recognition sequence and second recognition sequence. That is, in this double-stranded DNA fragment the tag sequence protrudes as a single strand on the 5' side of one DNA strand, and the labeling-binding region protrudes on the 5' side of the other DNA strand.

When the second primer has a label bound in advance to the label-binding region as shown in FIG. 2A, this results in a double-stranded DNA fragment having the label at the 5' end of one DNA strand and the tag sequence protruding on the 5' end of the other DNA strand, together with a double strand that includes the first and second recognition sequences.

As shown in FIG. 2C, moreover, this results in a double-stranded DNA fragment having a label at a DNA strand elongation site and a tag sequence protruding on the 5' side of one DNA strand, with the first and second recognition sequences included in a double strand.

A BSA or other water-soluble polymer and a magnesium salt may be contained in the nucleic acid amplification reaction solution. Therefore, the BSA or other water-soluble polymer and magnesium salt contained in the nucleic acid amplification reagent or nucleic acid amplification reaction solution may serve to replenish part of the water-soluble polymer and magnesium salt in the chromatography liquid. In this case, the contents of the water-soluble polymer and magnesium salt may be adjusted as necessary to form the composition of the chromatography liquid.

The target nucleic acid may be labeled in advance with a label as discussed below. The label may also be incorporated directly in the nucleic acid amplification reaction, or by an enzyme reaction for example. Even without this, however, a labeling step may be performed separately to form a hybridization product between an amplification product or other target nucleic acid and a labeled probe labeled with a label capable of hybridizing with the target nucleic acid. Alternatively, a hybridization product can be formed with an unlabeled target nucleic acid in the chromatography liquid as follows.

The chromatography liquid may contain a label for recognizing the target nucleic acid. When the label is included in the chromatography liquid, there is no need to label the target nucleic acid on the chromatography body after supplying the chromatography liquid containing the target nucleic acid to the chromatography body, nor is it necessary to provide a complexing site on the chromatography body for forming a complex between the target nucleic acid and the label. It is also possible to omit the operation of attaching the label to the amplification product in the nucleic acid amplification step for preparing the target nucleic acid.

In the Description, a "label" is a substance that allows a substance or molecule that is an object of detection to be distinguished from others. The label is not particularly limited, but typical examples include labels using fluorescence, radioactivity, enzymes (for example, peroxidase, alkaline phosphatase, etc.), hapten (digoxigenin (DIG), etc.), phosphorescence, chemoluminescence, coloration and the like. A label in the Description may be a molecule or substance capable of binding this so as to allow ultimate recognition by the label.

The label is preferably a luminescent substance or chromogenic substance that presents luminescence or coloration that is detectable with the naked eye. That is, it is preferably a substance that can produce a signal that is itself directly visible to the naked eye, without the need for another component. Typical examples of such substances include various pigments, dyes and other colorants. Equivalent substances include gold, silver and other precious metals, and copper and various other metals and alloys, as well as organic compounds containing these metals (which may be complex compounds). Mica and other inorganic compounds are also equivalent to colorants.

Typical examples of such labels include various dyes and pigments and chemoluminescent substances including luminol, isoluminol, acridinium compounds, olefins, enol ethers, enamines, aryl vinyl ethers, dioxenes, aryl imidazole, lucigenin, luciferin and aequorin. Other examples include latex or other particles labeled with these labels. Other examples include colloids or sols including gold colloids or sols and silver colloids or sols. Metal particles, inorganic particles and the like are also possible.

The average particle diameter of latex particles constituting part of the label is not particularly limited, but the mean particle diameter may be 20 nm to 20 µm for example, and is typically 40 nm to 10 µm, or preferably 0.1 µm to 10 µm, or more preferably 0.1 µm to 5 µm, or still more preferably 0.15 µm to 2 µm. Preferred are particles made of a water-insoluble polymer material that can be suspended in an aqueous solution. Examples include polyethylene, polystyrene, styrene-styrene sulfonate copolymer, acrylic acid polymers, methacrylic acid polymers, acrylonitrile polymers, acrylonitrile-butadiene-styrene, polyvinyl acetate-acrylate, polyvinyl pyrrolidone or vinyl chloride-acrylate. Latex particles of such polymers having surface carboxyl, amino or aldehyde groups or other active groups are also possible.

Such a label may be obtained commercially, but methods for manufacturing labels and methods of labeling particles are well known, so the label can be obtained by a person skilled in the art using known techniques as necessary. Moreover, binding between such a label or a particle labeled with such a label and an oligonucleotide such as DNA can be accomplished appropriately via an amino or other functional group, and these matters are well known in the field.

The label contained in the chromatography liquid may take the following form for example. It may be in the form of a labeling probe that is provided with a label and is capable of hybridizing with the target nucleic acid. For instance, the labeling probe may be provided with a label and a labeling sequence that hybridizes with a part of the target nucleic acid amplification product (the labeling tag sequence). The labeling sequence is complementary to a degree that allows it to hybridize specifically with the labeling tag sequence, and is preferably entirely complementary. The base sequence length of the labeling sequence is not particularly limited, and is determined appropriately according to the nature of the target sequence of the target nucleic acid and the Tm and the like associated with hybridization. A preferred label of a labeling probe is a label that emits color or fluorescence detectable with the naked eye in the probe region, and more preferably it is one or two or more selected from the gold colloid particles, silver colloid particles and colored latex particles. Using such a labeling probe, it is possible to detect a target nucleic acid in situ without using a fluorescence measurement device. Because the chromatography liquid of the invention contains a water-soluble polymer, moreover, stable detection sensitivity can be obtained when such particles are used.

The steps for performing chromatography using the chromatography body and chromatography liquid explained above are explained next.

When performing the chromatography step, the embodiment of the nucleic acid chromatography is not particularly limited. It may be designed for development in a roughly horizontal state. In this case, chromatography is typically performed by dripping or otherwise applying a specific amount of the chromatography liquid to a specific chromatography liquid supply part. The form of chromatography may also be designed for development in a roughly perpendicular state. In this case, chromatography is typically performed by supporting the chromatography body in a roughly perpendicular direction and immersing the lower part of the body in the chromatography liquid.

When the chromatography liquid is supplied to the chromatography body according to the mode of chromatography, the chromatography liquid disperses the solid-phase carrier and expands to the probe region. When the label is not supplied to the target nucleic acid, the target nucleic acid binds to a label provided in advance in a complexing part upstream from the probe region, forming a complex with the label and expanding to the probe region.

When a detection probe for hybridization with the target nucleic acid is fixed to the probe region, the target nucleic acid hybridizes with that detection probe, forming a hybridization product. This allows the provision of a signal whereby the target nucleic acid can be detected according to the kind of label attached to the target nucleic acid.

Because a water-soluble polymer is contained in the chromatography liquid in the chromatography method of the invention, the concentration of the target nucleic acid in the probe region is increased, and detection of the target nucleic acid is stabilized. Detection is also made simultaneously more sensitive and more rapid. For example, a target nucleic acid can be detected in as little as 5 minutes when a water-soluble polymer and a magnesium salt are included.

When there are multiple probe regions, if there is a detection probe designed to hybridize with another target nucleic acid in the hybridization solution, a hybridization product is formed in that probe region.

The conditions in the chromatography step are not particularly limited, but for example chromatography may be performed in an air atmosphere at about 5°C to 40°C, or preferably 15°C to 35°C. Chromatography is initiated for example by saturating a part (lower part or supply part) of a sheet-shaped chromatography body 2.0 mm to 8.00 mm in width and 20 mm to 100 mm in length (or height) with about 10 µl to 60 µl of the chromatography liquid. The developing time required for the chromatography liquid to complete passage through the probe region is 2 minutes to 50 minutes.

### (Detection step)

The detection step is a step of detecting a final hybridization product based on the label. More specifically, it is a step of confirming the coloring and position of a fixing region with a detection probe fixed thereon. The means of detecting the signal produced by the label is selected appropriately according to the type of label. When a specific binding reaction or enzyme coloring reaction is required, such operations are performed appropriately. In the chromatography method of the invention, the detection step is preferably performed as is without washing the solid-phase carrier.

When the label is one such as latex particles, gold colloid particles or silver particles that emits coloration or light that is visible to the naked eye, the presence and amount of the target nucleic acid can be detected directly with the naked eye (from the color intensity or the like). This allows for even more rapid detection.

### (Nucleic acid chromatography composition and kit)

The composition disclosed in the Description contains one or two or more kinds of water-soluble polymers selected from the group consisting of albumin, casein, skim milk, globulin, gelatin, heparin, polyethylene glycol, polyvinylpyrrolidone, dextran, polyvinyl alcohol, carboxymethyl cellulose and polymaleimide, and is a composition for preparing a chromatography liquid to be used in nucleic acid chromatography.

The chromatography composition may be provided in advance with a composition corresponding to the final concentration of the chromatography liquid (first embodiment).

The chromatography composition may also be in the form of a mix-on-demand powder or solids with a composition that allows the chromatography liquid to be prepared by dissolving the powder or solids in water or a buffer solution optionally containing some of the components of the chromatography liquid as needed (second embodiment).

The chromatography composition may also be in the form of a concentrated solution (stock solution) that can be diluted with water or buffer solution to prepare the chromatography liquid of the invention (third embodiment). In this case, a kit may be configured from this composition and an aqueous medium such as water or buffer solution for diluting the composition.

The chromatography composition may also be in the form of a stock solution with a composition that allows the chromatography liquid of the invention to be prepared by mixing the composition with a specific amount of an amplification reaction solution from a PCR amplification reaction (fourth embodiment). In this case, a kit may be configured from this composition, an aqueous medium such as water or buffer solution for diluting the composition, and an amplification reaction reagent.

This chromatography composition may also be made into a kit provided with either or both of the chromatography body explained above and a label of one of the various embodiments.

The concentration of the water-soluble polymer in the chromatography composition of the third embodiment may be for example 0.2 mass% to 6.0 mass% considering the concentration of the water-soluble polymer in the chromatography liquid. The concentration of the magnesium salt in the chromatography composition is preferably 1.0 mM to 20.0 mM. The concentration of the surfactant in the chromatography composition is preferably 0.1 mass% to 0.5 mass%. This is because the composition can be suitably diluted 1.4 to 2.0 times.

The concentration of the water-soluble polymer in the chromatography composition of the fourth embodiment can similarly be 0.1 mass% to 5.0 mass%. The concentration of the magnesium salt in this chromatography composition is preferably 0.5 mM to 16.0 mM. The concentration of the surfactant in this chromatography composition is preferably 0.1 mass% to 0.5 mass%. This is because the composition can suitably be diluted 1.4 to 2.0 times with the nucleic acid amplification reaction solution. In the chromatography composition of the fourth embodiment, the water-soluble polymer concentration and magnesium salt concentration are adjusted appropriately according to the BSA or other water-soluble polymer concentration and the magnesium salt concentration of the amplification reagent used.

### [Examples]

The present invention is explained in detail below with examples, but these examples are provided to explain the present invention and do not limit the same. In the following examples, all percentages (%) represent mass percentages.

### [Example 1]

(Detection 1 of target nucleic acid by nucleic acid chromatography using chromatography liquid comprising an amplification reaction solution containing a target nucleic acid as an amplification product)

PCR amplification was performed on normal human tissue genome DNA using a commercial PCR amplification reagent with a primer set for obtaining partially double-stranded DNA. This was then supplied to nucleic acid chromatography. The test materials and methods were as follows. Partially double-stranded DNA comprising single strands at the 5' ends of both strands of the double strand is obtained with this primer set. Therefore, the target nucleic acid was supplied to nucleic acid chromatography without a heat-denaturing step.

### (1) Test materials

Genome DNA: Cosmo Bio normal human tissue genome DNA (Cat. No. D1234148)
PCR amplification reagent: Qiagen Multiplex PCR Kit (Cat. No. 206143)
Primers (forward, reverse): Synthetic primers consigned at Nihon Gene Research Laboratories

### (2) Primer set

Primer (Forward): 
5'-
(TGTTCTCTGACCAATGAATCTGCXACCAAAGAATATGGCTGAATTTAGTAGTGTTTT
AAATAATTTTAA)-3' (SEQ ID NO:101)
Primer (Reverse): 
5'-
(TTTTTTTTTTTTTTTTTTTTXACCTGCTAATGAGATGATCCCTTATTTTGAAAACAACT
ATTCCTA)-3' (SEQ ID NO:102)
Linking site X=Spacer C3 has the following structure.

[C4]

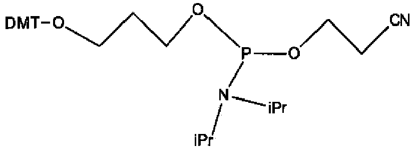

### (3) PCR reaction conditions

Reaction conditions: 95°C, 15 min, [95°C, 30 sec, 80°C, 1 sec, 64°C, 6 min] x 40 cycles

Composition of PCR reaction solution:

**[Table 1]**

| Reagent | Content |
|---|---|
| 2x Qiagen Multiplex PCR Master Mix *1 | 5.0 µl |
| STH amplification primers (500 nM each) | 0.5 µl |
| RNase free water | 2.0 ul |
| Genome DNA (10 ng/µl) | 2.5 µl |
| Total content | 10.0 µl |

| | |
|---|---|
| *1: MgCl₂ (magnesium chloride) final concentration: 3 mM | |

### (4) Preparation of nucleic acid chromatography body

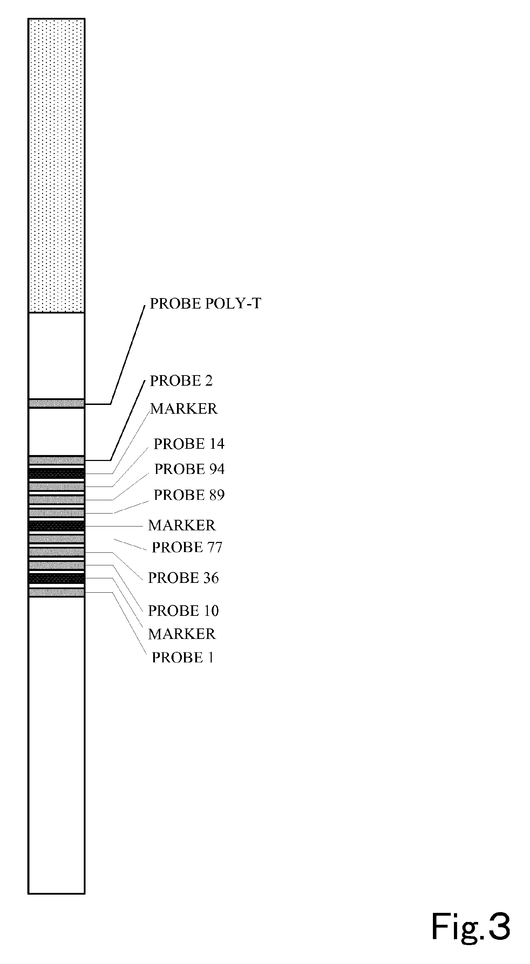

Three marker sites using red pigment and probes having the eight different base sequences represented by SEQ ID NOS:1, 2, 10, 14, 36, 77, 89 and 94 (Probes 1, 2, 10, 14, 36, 77, 89 and 94) were spotted on a Merck Millipore membrane sheet (Hi-Flow Plus HF 180) (width 3.5 mm, length 60 mm) in the pattern shown in FIG. 3, using a NGK Insulators, Ltd. Geneshot® spotter, to form a total of eight band-shaped probe regions. A poly-T probe was also spotted further downstream. Probe 1 targets the amplification product obtained in (3).

### (5) Preparation of nucleic acid chromatography composition

Developing solutions 1 to 4 each containing a surfactant, a phosphate buffer (pH 7.4) and bovine serum albumin (BSA) were prepared as chromatography compositions (developing solutions). For the label, a 2.5% suspension of color latex (blue) particles with attached tag DNA (poly-A) was prepared as a colorant.
Developing solution 1: 0.1% Tween 80-phosphate buffer
Developing solution 2: 0.1% Tween 80-0.5% BSA-phosphate buffer
Developing solution 3: 0.1% Tween 80-1.0% BSA-phosphate buffer
Developing solution 4: 0.1% Tween 80-3.0% BSA-phosphate buffer

Tween 80: Wako Pure Chemical Industries, Ltd. Polyoxyethylene (20) Sorbitan Monooleate (Cat. No. 163-21625)
BSA: Roche Diagnostics albumin fraction V (Cat. No. 735086)
Phosphate buffer: Mixed solution of disodium hydrogen phosphate dodecahydrate and potassium dihydrogenphosphate

### (6) Nucleic acid chromatography

Using all 10 µl of the PCR amplification reaction solution obtained in (3), 50 µl each of chromatography liquids 1 to 4 were prepared with the following compositions. In chromatography liquids 1 to 4, the concentration of the magnesium salt from the PCR amplification product was 0.6 mM. The BSA concentrations of chromatography liquids 1 to 4 were 0%, 0.35%, 0.7% and 2.1%, respectively.

**[Table 2]**

| Reagent | Content | Final concentration (salt) |
|---|---|---|
| PCR amplification product (3 mM MgCl₂) | 1.0 ul | 0.6 mM MeCl₂ |
| Colorant | 5 ul | |
| Developing solutions (1 to 4) *2 | 35 ul | |
| Total content | 50 ul | |

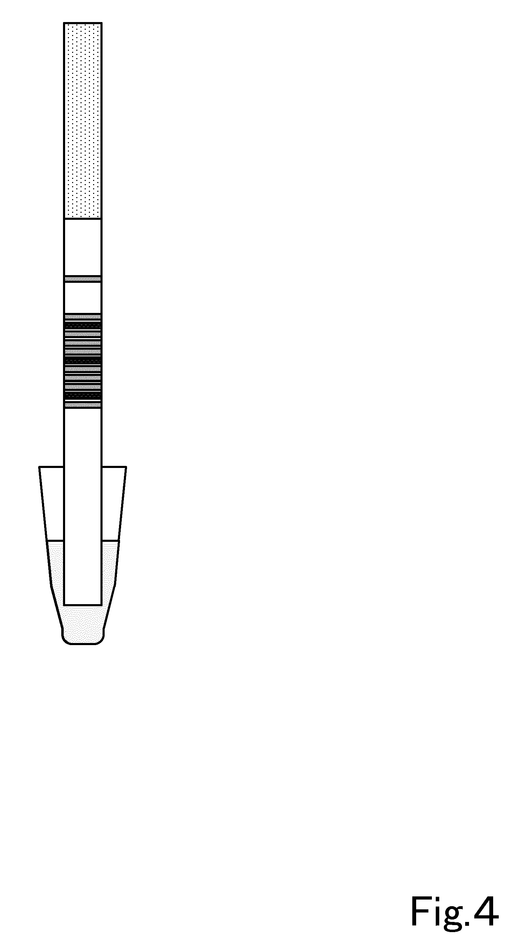

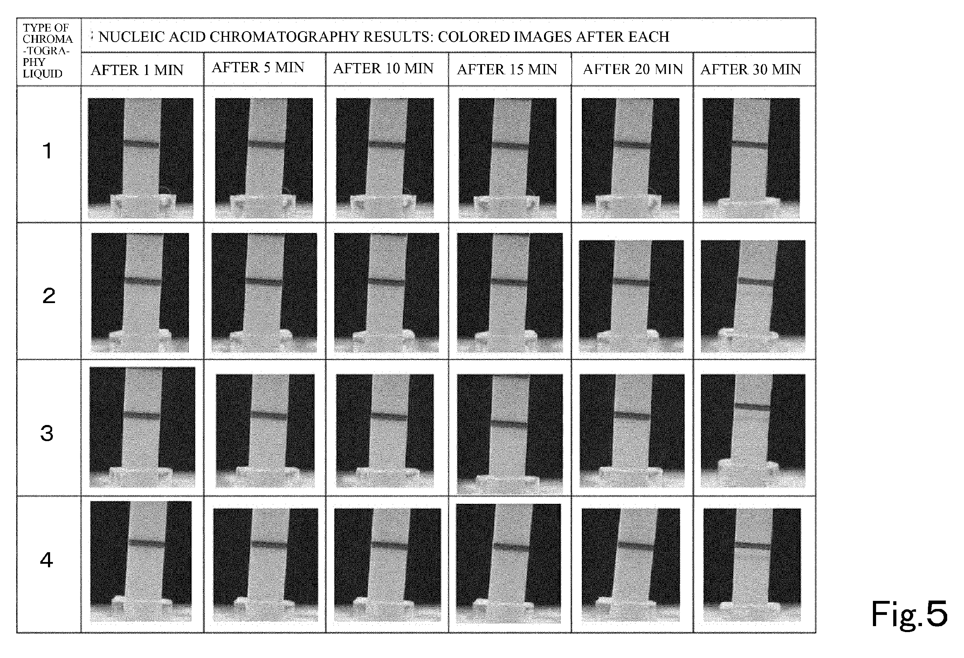

As shown in FIG. 4, 50 µl of each chromatography liquid was added to a microtube, and the lower end of the chromatography body was immersed in the chromatography liquid until all of the chromatography liquid was taken up (about 20 minutes), after which this was dried as is for 10 minutes. The results are shown in FIG. 5.

As shown in FIG. 5, with the chromatography liquid 1 containing no BSA, almost no colored band appeared in the probe region of probe 1 even after 30 minutes. By contrast, with chromatography liquids 2 to 4 containing 0.35 to 2.1 mass% BSA, obvious colored bands appeared in the probe region of probe 1 after 20 minutes. The clearest band was observed with the chromatography liquid 4, which had the highest concentration of BSA.

### [Example 2]

### (Detection 2 of target nucleic acid by nucleic acid chromatography using chromatography liquid comprising an amplification reaction solution containing a target nucleic acid as an amplification product)

The same operations were performed as in Example 1 using a different commercial PCR amplification reagent, PCR reaction conditions and PCR reaction solution composition from Example 1, and the result was supplied to nucleic acid chromatography. The PCR amplification reagent, reaction conditions and the like were as follows.
PCR amplification reagent: Nippon Genetics Co. KAPA2G Fast HotStart PCR Kit (Cat. No. KK5502)
PCR reaction conditions: 95°C, 3 minutes, [95°C, 15 sec, 68°C, 2 min] x 40 cycles
PCR reaction solution composition:

**[Table 3]**

| Reagent | Content |
|---|---|
| 5xKAPA2B Buffer *1 | 3.0 µl |
| STH Amplification primers (500 nM each) | 0.5 ul |
| dNTP Mixture (10 mM each) | 0.2 µl |
| MgCl₂ (25 mM) *1 | 0.9 µl |
| DNA polymerase (5U/µl) | 0.08 ul |
| RNase free water | 2.82 ul |
| Genome DNA (10 ng/µl) | 2.5 ul |
| Total content | 10.0 µl |

| | |
|---|---|
| *1: Final concentration of MgCl₂ (magnesium chloride): 4.5 mM | |

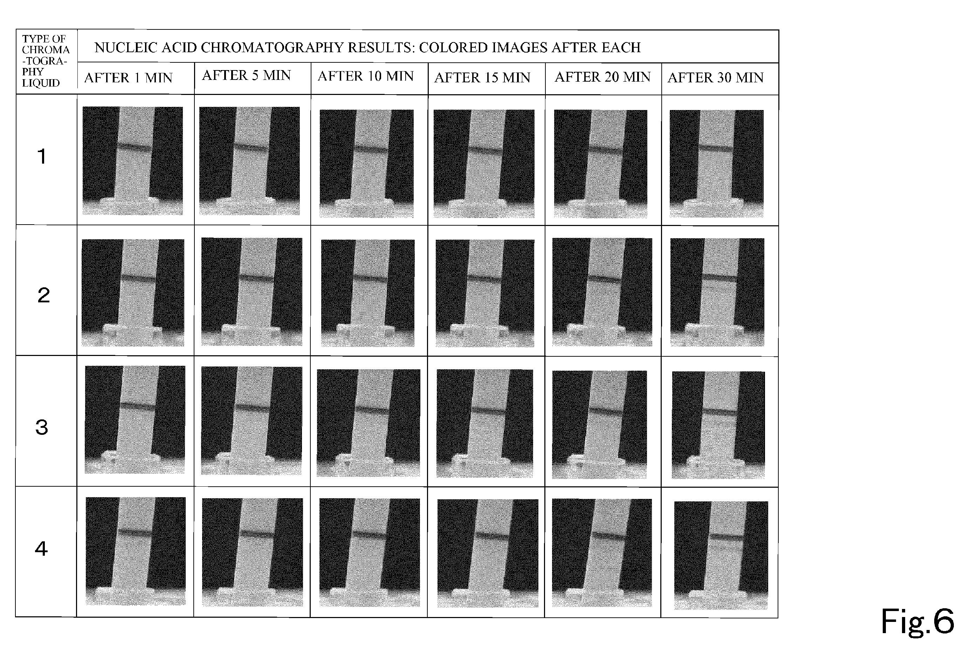

In the chromatography liquids 1 to 4 of Example 2, the concentration of magnesium salt derived from the PCR amplification product was 0.9 mM. The BSA concentrations of the chromatography liquids 1 to 4 were 0%, 0.35%, 0.7% and 2.1%, respectively. The results are shown in FIG. 6.

As shown in FIG. 6, with the chromatography liquid 1 containing no BSA, almost no colored band appeared in the probe region of probe 1 even after 30 minutes as in Example 1. By contrast, with the chromatography liquids 2 to 4 containing 0.35 to 2.1 % BSA, obvious colored bands appeared in the probe region of probe 1 after 15 minutes. The clearest band was observed with the chromatography liquid 4, which had the highest concentration of BSA. Because the concentration of the magnesium salt was higher than in Example 1 (0.9 mM) in chromatography liquids 2 to 4, a blue band began to be detected after 10 minutes.

### [Example 3]

### (Evaluation of salts in chromatography liquid obtained from nucleic acid chromatography using synthetic oligo-DNA)

Using synthetic oligo-DNA as the target nucleic acid, the relationship between the type of salt in the chromatography liquid and the level of detection of the target nucleic acid was evaluated. The same operations were performed as in Example 1 using synthetic oligo-DNA of the following sequence as the target nucleic acid, except that no amplification reaction was performed with the DNA, and chromatography liquids 1 to 3 were prepared with the following compositions and supplied to nucleic acid chromatography. The base sequence of the synthetic oligo-DNA and the compositions of the chromatography liquids were as follows. The poly-T part is a site for hybridization with the DNA tag (poly-A) of the colored latex particles.

### Synthetic oligo-DNA (complement oligo): synthetic product from Nihon Gene Research Laboratories

### Synthetic oligo-DNA data (sequence)

Complement oligo-DNA (Probe No. 1): 5'-(TTTTTTTTTTTTTTTTTTTTTGTTCTCTGACCAATGAATCTGC)-3' (SEQ ID NO: 103)

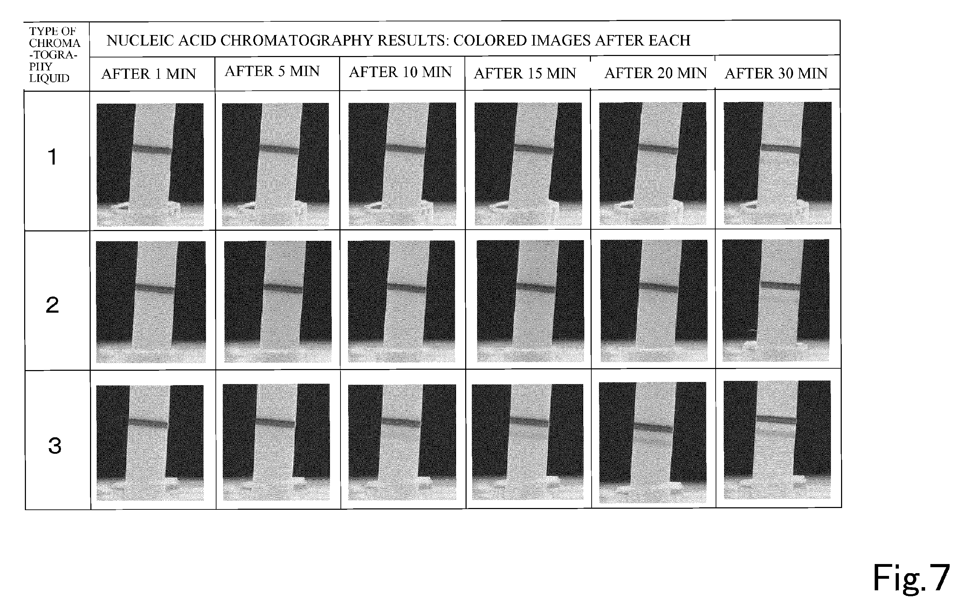

Chromatography liquids 1 to 3 were prepared according to the following table. Developing solution 4 was 0.1% Tween 80-3.0% BSA-phosphate buffer, and salts 1 to 3 were 25 mM sodium chloride (salt 1), 25 mM potassium chloride (salt 2) and 25 mM magnesium chloride (salt 3). The final concentration of each was 3 mM. The final concentration of the synthetic oligo-DNA was 100 nM, while that of BSA was 2.1 %. The results are shown in FIG. 7.

**[Table 4]**

| Reagent | Content | Final concentration |
|---|---|---|
| Synthetic oligo-DNA (5 µM) | 1 µl | 100 nM |
| Colorant | 5 µl | |
| 25 mM salt (1 to 3) *1 | 6 µl | 3 mM salt |
| Sterile water | 3 µl | |
| Developing solution 4 | 35 µl | |
| Total content | 50 µl | |

As shown in FIG. 7, when a magnesium salt was used (salt 3) a blue band was confirmed in the probe region of probe 1 after 5 minutes. Using the sodium salt (salt 1) and potassium salt (salt 2), the blue band was not observed only after 20 minutes. With the salt 3, the coloration of the blue band was roughly maximum after 20 minutes. These results show that unlike alkali salts, a magnesium salt serves to effectively concentrate the target nucleic acid in the probe region in the presence of a water-soluble polymer such as BSA, thereby contributing to highly sensitive and rapid verification of a target nucleic acid.

### [Example 4]

### (Evaluation of magnesium salt concentration in chromatography liquid from nucleic acid chromatography using synthetic oligo-DNA)

The same operations were performed as in Example 3 except that chromatography liquids were prepared with the following compositions, and nucleic acid chromatography was performed.

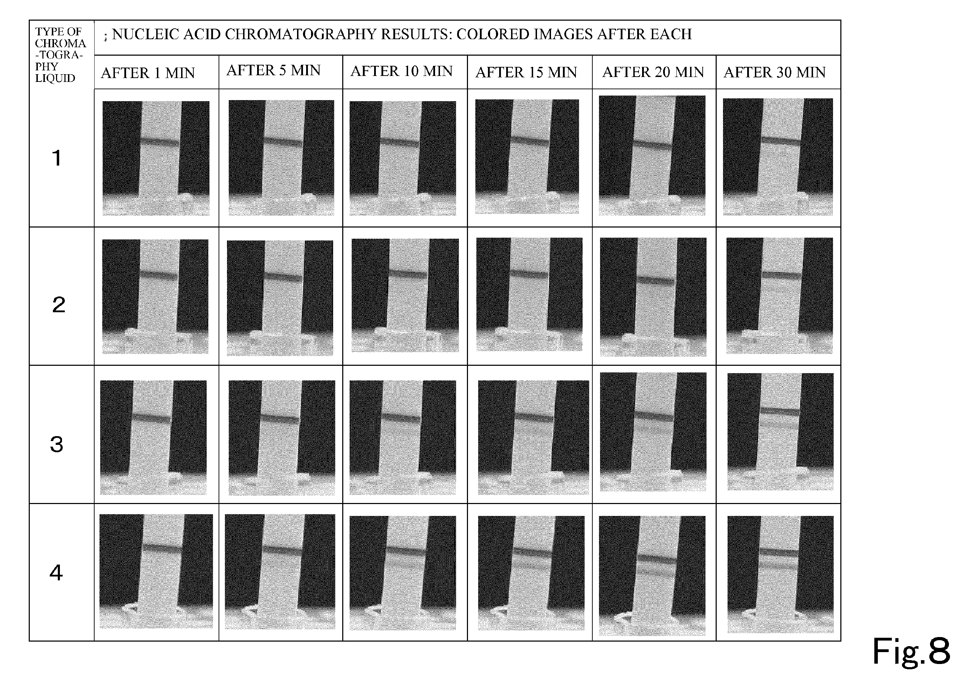

The chromatography liquids were prepared according to the following table. Developing solution 4 was 0.1 % Tween 80-3.0% BSA-phosphate buffer, and the salt was 25 mM magnesium chloride (salt 3), prepared to final concentrations of 0 mM, 3 mM and 4.5 mM. The final concentration of the synthetic oligo-DNA was 100 nM, and that of BSA was 2.1%. The results are shown in FIG. 8.

**[Table 5]**

| Reagent | Content | Final concentration |
|---|---|---|
| Synthetic oligo-DNA (5 µM) | 1 µl | 100 nM |
| Colorant | 5 µl | |
| 25 mM magnesium chloride (MgCl₂) *1 | Xµl | 0 to 4.5 mM MgCl₂ |
| Sterile water | Yµl | |
| Developing solution 4 | 35 µl | |
| Total content | 50 µl | |

As shown in FIG. 8, more sensitive and rapid detection was possible the higher the concentration of the magnesium salt. That is, when the magnesium concentration was 3 mM and 4.5 mM, obvious blue bands were confirmed 5 minutes after the start of development. Even at a concentration of 1 mM, a blue band was confirmed after 10 minutes. These results show that concentration of the target nucleic acid on the detection probe is promoted without inhibiting hybridization between the target nucleic acid and the detection probe even at a relatively high magnesium concentration.

### [Example 5]

### (Detection 3 of target nucleic acid by nucleic acid chromatography using chromatography liquid comprising an amplification reaction solution containing a target nucleic acid as an amplification product)

The same operations were performed as in Example 1 except that developing solutions 4 to 7 were used instead of the developing solutions 1 to 4 of Example 1, and chromatography liquids were prepared with the final concentration of total magnesium salts adjusted to 0.6 mM, 4.1 mM, 8.3 mM and 16.7 mM, and supplied to nucleic acid chromatography. The compositions of the newly prepared developing solutions 5, 6 and 7 were as follows.
Developing solution 5: 0.1% Tween 80-3.0% BSA-5.0 mM magnesium chloride-phosphate buffer
Developing solution 6: 0.1% Tween 80-3.0% BSA-11.0 mM magnesium chloride-phosphate buffer
Developing solution 7: 0.1% Tween 80-3.0% BSA-23.0 mM magnesium chloride-phosphate buffer

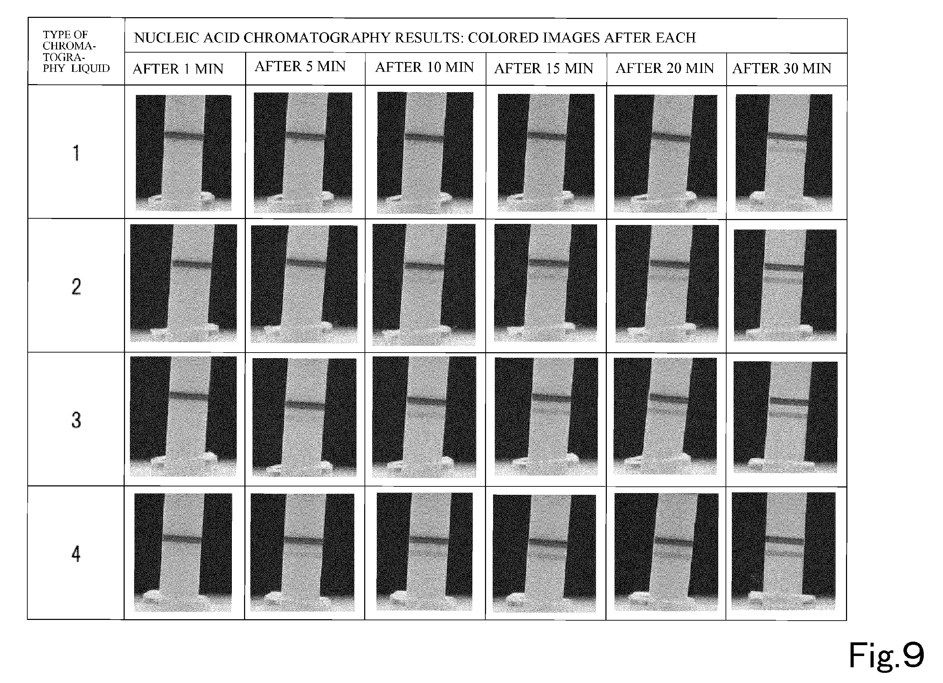

In the chromatography liquids 1 to 4 of Example 4, the concentration of magnesium salts derived from the PCR amplification product was 0.6 mM. The concentrations of magnesium chloride separately derived from the developing solutions were 3.5 mM, 7.7 mM and 16.1 mM, so the total magnesium chloride concentrations in the chromatography liquids 1 to 4 were 0.6 mM, 4.1 mM, 8.3 mM and 16.7 mM. The BSA concentrations of chromatography liquids 1 to 4 were all 2.1%. The results are shown in FIG. 9.

As in Example 4, more sensitive and rapid detection was possible at higher magnesium salt concentrations, as shown in FIG. 9. That is, obvious blue bands were observed 5 minutes after the start of development with magnesium concentrations of 4.1 mM and 8.3 mM. At 0.6 mM, a light blue band was finally confirmed after 20 minutes. At 16.7 mM, on the other hand, the target bands were strongly colored blue in each case, but after 30 minutes cross-hybridization occurred and there was blue coloration of non-target bands. These results show that concentration of the target nucleic acid on the detection probe can be promoted without inhibiting hybridization between the target nucleic acid and the detection probe by including magnesium chloride in the presence of a water-soluble polymer such as BSA even in the case of a PCR amplification reaction solution or the like, in which residual DNA polymerase and other enzymes, primers, nucleotides and the like are present in addition to the amplification product (target nucleic acid). Considering cross-hybridization, the magnesium salt concentration is preferably lower than 16.7 mM.

### [Example 6]

### (Detection 4 of target nucleic acid by nucleic acid chromatography using chromatography liquid comprising an amplification reaction solution containing a target nucleic acid as an amplification product)

The same operations were performed as in Example 2 except that developing solutions 4 to 7 were used instead of the developing solutions 1 to 4 used in Example 2, and chromatography liquids were prepared with final concentrations of magnesium chloride in total of 0.9 mM, 4.4 mM, 8.6 mM and 17.0 mM, and supplied to nucleic acid chromatography. The compositions of the newly prepared developing solutions 5, 6 and 7 were as follows.
Developing solution 5: 0.1% Tween 80-3.0% BSA-5.0 mM magnesium chloride-phosphate buffer
Developing solution 6: 0.1% Tween 80-3.0% BSA-11.0 mM magnesium chloride-phosphate buffer
Developing solution 7: 0.1% Tween 80-3.0% BSA-23.0 mM magnesium chloride-phosphate buffer

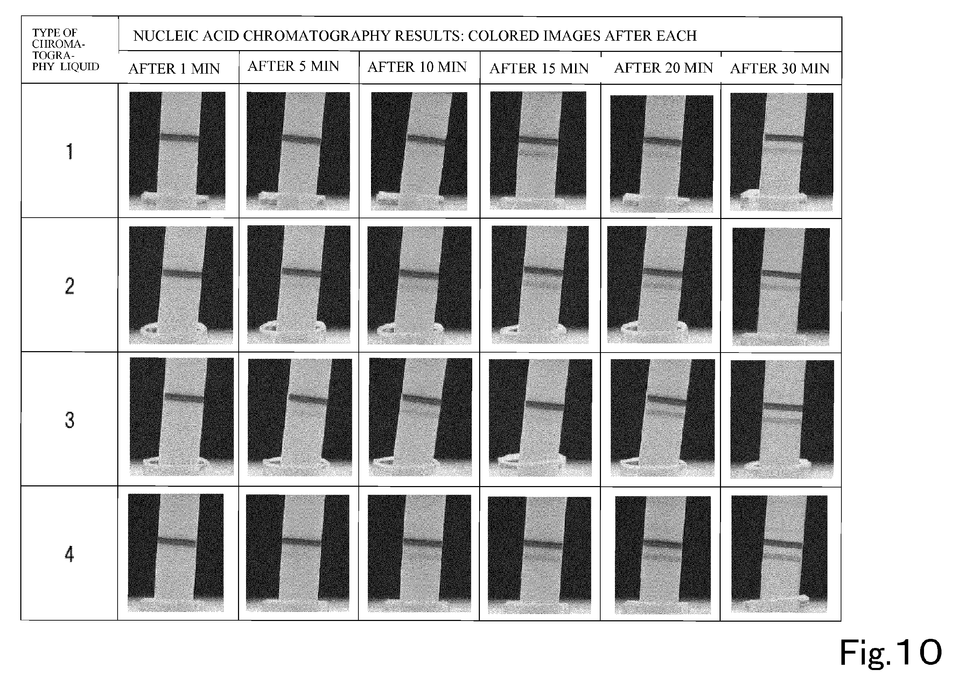

In the chromatography liquids 1 to 4 of Example 6, the concentration of magnesium salts derived from the PCR amplification product was 0.9 mM. The concentrations of magnesium chloride separately derived from the developing solution were 3.5 mM, 7.7 mM and 16.1 mM, so the total magnesium chloride concentrations of the chromatography liquids 1 to 3 were 0.9 mM, 4.4 mM, 8.6 mM and 17.0 mM. The BSA concentrations of the chromatography liquids 1 to 4 were all 2.1 %. The results are shown in FIG. 10.

As in Example 5, more sensitive and rapid detection was possible at higher magnesium salt concentrations, as shown in FIG. 10. That is, obvious blue bands were observed 5 minutes after the start of development with magnesium concentrations of 4.4 mM and 8.6 mM. In particular, a blue band was confirmed after the elapse of 5 minutes with the 8.6 mM concentration. At 0.9 mM, a light blue band was finally confirmed after 15 to 20 minutes. At 17.0 mM, on the other hand, the target band was strongly colored blue, but after 20 minutes cross-hybridization occurred and there was blue coloration of non-target bands. Considering cross-hybridization, this shows that the magnesium salt concentration is preferably less than 17.0 mM.

These results show that concentration of the target nucleic acid on the detection probe can be promoted without inhibiting hybridization between the target nucleic acid and the detection probe by including magnesium chloride in the presence of a water-soluble polymer such as BSA even in the case of a PCR amplification reaction solution or the like, in which residual DNA polymerase and other enzymes, primers, nucleotides and the like are present in addition to the amplification product (target nucleic acid).

### [Sequence List Free Text]

SEQ ID NOS:1 to 100, 103: probes, SEQ ID NOS:101 and 102: primers

## Claims

1. A nucleic acid chromatography method, comprising a step of performing chromatography by supplying a chromatography liquid containing a target nucleic acid to a solid-phase carrier on which a detection probe capable of capturing the target nucleic acid is fixed, wherein
the chromatography liquid contains one or two or more kinds of water-soluble polymers selected from the group consisting of albumin, casein, skim milk, globulin, gelatin, heparin, polyethylene glycol, polyvinylpyrrolidone, dextran, polyvinyl alcohol, carboxymethyl cellulose and polymaleimide.

2. The method according to Claim 1, wherein the water-soluble polymer includes albumin.

3. The method according to Claim 1 or 2, wherein a concentration of the water-soluble polymer in the chromatography liquid is 3.5 mass% or less.

4. The method according to any one of Claims 1 to 3, wherein the chromatography liquid further contains magnesium salt.

5. The method according to Claim 4, wherein the magnesium salt includes magnesium chloride.

6. The method according to Claim 4 or 5, wherein a concentration of the magnesium salt is 0.5 mM or more and 10.0 mM or less.

7. The method according to any one of Claims 1 to 6, wherein the chromatography liquid further contains a surfactant.

8. The method according to any one of Claims 1 to 7, wherein the chromatography liquid further contains a label for distinguishing the target nucleic acid.

9. The method according to Claim 8, wherein the label is colored particles.

10. The method according to any one of Claims 1 to 9, further comprising a step of preparing the chromatography liquid prior to the chromatography step,
the chromatography liquid including a nucleic acid amplification reaction solution containing the target nucleic acid as an amplification product.

11. The method according to any one of Claims 4 to 6, wherein at least a part of the magnesium salt is replenished by magnesium salt in the nucleic acid amplification reaction solution.

12. A composition for nucleic acid chromatography,
the composition containing one or two or more kinds of water-soluble polymers selected from the group consisting of albumin, casein, skim milk, globulin, gelatin, heparin, polyethylene glycol, polyvinylpyrrolidone, dextran, polyvinyl alcohol, carboxymethyl cellulose and polymaleimide.

13. The composition according to Claim 12, wherein the water-soluble polymer includes albumin.

14. The composition according to Claim 12 or 13, containing the water-soluble polymer so that a final concentration of the water-soluble polymer is 3.5 mass% or less.

15. The composition according to any one of Claims 12 to 14, further containing magnesium salt.

16. The composition according to Claim 15, wherein the magnesium salt includes magnesium chloride.

17. The composition according to any one of Claims 12 to 16, containing the magnesium salt so that a final concentration of the magnesium salt is 0.5 mM or more and 10.0 mM or less.

18. The composition according to any one of Claims 12 to 17, further containing a surfactant.

19. The composition according to any one of Claims 12 to 18, the composition being configured to be added to a nucleic acid amplification reaction solution containing an amplification product corresponding to a target nucleic acid.

20. A kit for nucleic acid chromatography,
the kit comprising a composition for nucleic acid chromatography containing one or two or more kinds of water-soluble polymers selected from the group consisting of albumin, casein, skim milk, globulin, gelatin, heparin, polyethylene glycol, polyvinyl pyrrolidone, dextran, polyvinyl alcohol, carboxymethyl cellulose and polymaleimide.

21. The kit according to Claim 20, wherein the composition for nucleic acid chromatography further contains magnesium salt.
